# EUROPEAN PATENT SPECIFICATION

(11) **EP 3 043 778 B1**
(45) Date of publication and mention of the grant of the patent: **06.09.2017**
(21) Application number: 14766156.5
(22) Date of filing: 11.09.2014
(51) Int. Cl.: A61K 9/28, A61K 31/18, A61K 9/16, A61K 9/20

(54) **PHARMACEUTICAL COMPOSITIONS CONTAINING REFAMETINIB**
PHARMAZEUTISCHE ZUSAMMENSETZUNGEN ENTHALTENDES REFAMETINIB
COMPOSITIONS PHARMACEUTIQUES CONTENANT DU REFAMETINIB

(30) Priority: 13.09.2013 EP 13184229; 16.09.2013 EP 13184539
(43) Date of publication of application: 20.07.2016
(73) Proprietor: Bayer Pharma Aktiengesellschaft, 13353 Berlin (DE)
(72) Inventor: STROYER, Anke, 12159 Berlin (DE); SKRABS, Susanne, 50859 Köln (DE); FORMELL, Michael, 16548 Glienicke (DE); WINTER, Gabriele, 16548 Glienicke (DE); KISSENBECK, Rosemarie, 13503 Berlin (DE)
(74) Representative: HGF Limited
(86) International application number: PCT/EP2014/069453
(87) International publication number: WO 2015/036512

(56) References cited:
- WO-A1-2010/105110
- WO-A2-2012/120364
- US-A1- 2011 033 539

## Description

### Field of the Invention

The present invention relates to pharmaceutical compositions which contain refametinib, a hydrate, solvate, metabolite or pharmaceutically acceptable salt thereof or a polymorph thereof, and which are coated with a stability-maintaining coating, to processes of preparing such compositions, and to uses of such compositions for treating hyper-proliferative disorders and/or angiogenesis disorders, such as cancer, either as a sole agent or in combination with other anti-cancer therapies.

### Background of the Invention

Refametinib, which is (S)-N-(3,4-difluoro-2-(2-fluoro-4-iodophenylamino)-6-methoxyphenyl)-1-(2,3-dihydroxypropyl)cyclopropane-1-sulfonamide, of formula (I) 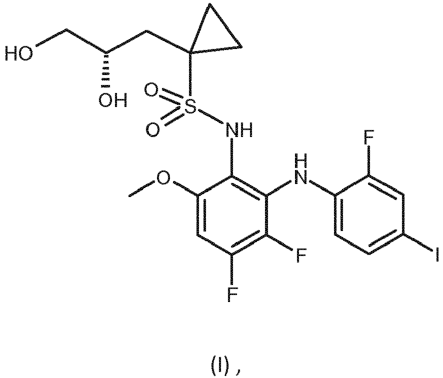 and also referred to as "BAY 86-9766" or "RDEA 119", is a highly potent and selective MEK1/2 inhibitor currently under development in clinical trials for treatment of late stage cancer patients refractory or intolerant to other anticancer therapies [*vide* C. Iverson, G. Larson, C. Lai, L.-T. Yeh, C. Dadson, P. Weingarten, T. Appleby, T. Vo, A. Maderna, J.-M. Vernier; R. Hamatake, J.N. Miner, B. Quart, in Cancer Res. 2009, 69, 6839-6847].

Refametinib as an amorphous compound is published in PCT application under WO 2007/014011 A2 and a crystalline form of refametinib (hereinafter referred to as "polymorph A") is published in PCT application under WO 2009/018233.

The initial synthesis of refametinib is published in US 2008/0058340 which comprises an osmium catalyzed dihydroxylation of the allyl sulfonamide substituted core followed by chromatographic separation of the enantiomers using a chiral stationary phase. Another synthesis of refametinib is published in a PCT patent application under WO 2011/009541 A1 as a chiral preparation of (R)- and (S)-N-{3,4-difluoro-2-[(2-fluoro-4-iodophenyl)amino]-6-methoxyphenyl}-1-[2,3-dihydroxy-propyl]cyclopropanesulfonamide and protected derivatives thereof. A chiral synthesis of refametinib is published in a PCT application under WO 2012/163799 making use of sodium 1-{[(4*S*)-2,2-dimethyl-1,3-dioxolan-4-yl]methyl}cyclopropanesulfonate.

### Pharmaceutical combinations of:

- refametinib and sorafenib are published in PCT application under WO 2009/018238 ;
- refametinib and gemcitabine are published in PCT application filed under PCT/US2010/27060.

Pharmaceutical compositions containing refametinib are published in a PCT application under WO 2009/129246, which are as formulations as follows :
- 20 mg refametinib dosage formulations :
   a) In re. 20 mg refametinib capsule formulations, WO 2009/129246 :
      - specifically exemplifies a 20 mg refametinib dosage formulation as a capsule in Example 102 ;
      - generically describes 20 mg refametinib dose capsules taking all excipients as carrier, in paragraph [0012] ;
      - generically claims 20 mg refametinib dosage formulation in claim 1; and
      - generically claims 20 mg refametinib dosage formulation as a capsule in claim 5.
   b) In re. 20 mg refametinib tablet formulations, WO 2009/129246 :
      - does not specifically exemplify 20 mg refametinib dosage formulations as a tablet as an Example;
      - generically claims 20 mg refametinib dosage formulation in claim 1; and
      - generically claims 20 mg refametinib dosage formulation as a tablet in claim 5.
- 10 mg refametinib dosage formulations :
   a) In re. 10 mg refametinib capsule formulations, WO 2009/129246 :
      - specifically exemplifies 10 mg refametinib dosage formulation as a capsule in Example 95A;
      - generically describes 10 mg refametinib dosage formulation in description, e.g. paragraph [0008] and in paragraph [0012] taking all excipients as carrier; also in [0015], also in paragraphs [0018] and [0022] but with the R-isomer of refametinib ;
      - generically claims 10 mg refametinib dosage formulations in claim 10 ;
      - does not generically claim 10 mg refametinib dosage formulation as a capsule in a claim dependent upon claim 10 ;
   b) in re. 10 mg refametinib tablet formulations, WO 2009/129246 :
      - does not specifically exemplify 10 mg refametinib dosage formulation as a tablet as an Example;
      - does not generically describe 10 mg refametinib dosage formulation as a tablet;
      - does not generically claim 10 mg refametinib dosage formulation in the claims ;
      - does not generically claim 10 mg refametinib dosage formulation as a tablet in a claim dependent upon an independent claim ;

Despite the progress described above, there still remains a need for improved oral pharmaceutical compositions containing refametinib which are superior to currently known formulations of refametinib with regard to physical and mechanical properties (such as high hardness, resistance to crushing and resistance to deformation, low friability and brittleness, independent of the storage conditions), with regard to the dimensions of the formulation and the drug load. In addition, there is still a need for new pharmaceutical compositions with better swallowability, improved patient compliance and convenience and higher safety during handling. Furthermore, there remains a need for oral pharmaceutical compositions which display good physical and chemical stability properties, particularly compositions which avoid and/or reduce degradation of the active agent and/or of the pharmaceutical composition comprising the active agent and which avoid and/or reduce changes in tablet hardness, disintegration and drug dissolution. Suitable oral pharmaceutical compositions allow, among other things, the amount of active agent to remain at a therapeutically effective dose and the amount of degradation impurities to be kept to a minimum and the dissolution profile to remain constant during manufacture and/or storage. This is particularly relevant for manufacture when, for example, the active substance is sensitive to particular excipients or substances (e.g. water) and for storage, when for example, long term storage and/or storage under extreme conditions (e.g. storage at elevated temperatures and/or high humidity conditions) is necessary.

The oral pharmaceutical composition has to provide a plasma level of the active agent which is sufficient for an effective therapy. This is dependent on the solubility and the release behaviour of the active agent. In the case of a solid pharmaceutical composition the disintegration and/or dissolution properties and chemical and mechanical stability are of importance. In order to support a high compliance, the oral pharmaceutical composition preferably should not have to be taken in more than three times a day, the less the better, and in the case of a tablet the dimensions of the tablet should not be too large. With each administration the number of tablets taken in at the same time preferably should not exceed two. The dimensions of a tablet are dependent on the amount of the active agent needed for an effective therapy and the amounts of the excipients. Moreover, for refametinib tablet formulations, and coated tablets in particular, are preferred to capsule formulations due to the limited mechanical strength of hard capsules. Upon mechanical stress, hard capsules may break or open and the content (such as powder blend containing the drug substance) may be spilled posing a significant safety risk. Providing a coated tablet dusting during handling is prevented and the risk of accidental exposure to the drug substance is minimized.

The preferred route of drug administration is through the oral cavity. This route provides the greatest comfort and convenience of dosing. Tablets are preferred forms of pharmaceutical compositions for oral administration. In order to administrate solid formulations conveniently, a coating is often needed. Objective of a coating can be to provide a homogeneous appearance, to mask discoloration during storage, to add color for product identification, to mask a bad taste, to prevent dusting during handling, to prevent abrasion or friction of a tablet, to increase mechanical stability, to facilitate and give a more convenient feeling when swallowing the tablet, in particular when the dimensions are large, to provide light protection for the drug or to protect the drug against humidity.

It was thus desired to prepare refametinib dosage formulations as coated tablets, particularly containing 10, 20, 30 and 50 mg of refametinib.

It has surprisingly been found, and this constitutes the basis of the present invention, that refametinib immediate release tablets, which were coated with a standard non-functional HPMC coating (consisting of hydroxypropyl methyl cellulose (HPMC), polyethylene glycol (PEG) and colorants (titanium dioxide and ferric oxide)) in an aqueous film-coating process coated show insufficient stability. The coating was applied for different reasons, e.g. to provide a homogeneous appearance, to add a color for product identification, to reduce dusting during handling of the tablets and to facilitate swallowing. When comparing the stability data of these coated refametinib tablets with the uncoated tablet cores it became apparent that the stability of the tablets was impaired by application of the coating : a significant decrease in drug dissolution of the coated tablets was observed during storage.

Applying an alternative HPMC based coating system, (consisting of HPMC, talc and colorants (titanium dioxide and ferric oxide)), or an alternative PVA based coating system (Opadry™ II 85G32407 yellow, consisting of polyvinyl alcohol (partially hydrolyzed) [44% by weight of the total mixture], polyethylenglycol (PEG 3350) [12.4% by weight of the total mixture], lecithin (soya), ferric oxide, titanium dioxide and talc; or the lecithin-free variant thereof) in the aqueous film-coating process, surprisingly, no such impairment of the drug dissolution after storage was observed. Coated with this alternative HPMC coating or with the alternative PVA based coatings, the tablets show excellent long term stability with no decrease in drug dissolution.

### Description of the Invention

The present invention thus pertains to a pharmaceutical composition comprising refametinib which is the compound of the formula (I) : 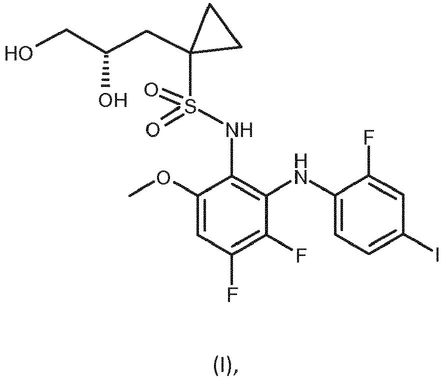 a hydrate, solvate, metabolite or pharmaceutically acceptable salt of refametinib, or a polymorph thereof and one or more pharmaceutically acceptable excipient wherein the pharmaceutical composition is coated by a stability-maintaining coating as defined herein, for example by an HPMC-based, polyethylene glycol-free, stability-maintaining coating or a polyvinyl alcohol (PVA)-based coating comprising 0 to 19% polyethylene glycol (PEG) by weight of said stability-maintaining coating and optionally one or more further pharmaceutically acceptable excipients.

In accordance with a first aspect, the present invention as defined in the claims relates :
* to coated tablets comprising :
   - a core, comprising :
      ∘ refametinib, a hydrate, solvate, polymorph, metabolite thereof, or a pharmaceutically acceptable salt thereof; and
      ∘ one or more pharmaceutically acceptable core excipients; and
   - a polyethylene glycol-free, stability-maintaining coating, comprising :
      ∘ a stability-maintaining film former, which is hydroxypropyl methylcellulose ;
      ∘ optionally, a further stability-maintaining film former;
      ∘ and, optionally,
      ∘ one or more pharmaceutically acceptable coating excipients.

In accordance with a second aspect, the present invention as defined in the claims relates :
* to coated tablets comprising :
   - a core, comprising :
      ∘ refametinib, a hydrate, solvate, polymorph, metabolite thereof, or a pharmaceutically acceptable salt thereof; and
      ∘ one or more pharmaceutically acceptable core excipients; and
   - a stability-maintaining coating, comprising :
      ∘ a stability-maintaining film former, which is polyvinyl alcohol, particularly partially hydrolysed polyvinyl alcohol ;
      ∘ optionally, a further stability-maintaining film former;
      ∘ a plasticizer, which is polyethylene glycol ;
      ∘ and, optionally,
      ∘ one or more pharmaceutically acceptable coating excipients.

The pharmaceutical compositions according to the present invention can be utilized to achieve the desired pharmacological effect by administration to a patient in need thereof. A patient, for the purpose of this invention, is a mammal, including a human, in need of treatment for the particular condition or disease. Therefore, the present invention includes pharmaceutical compositions which are comprised of a pharmaceutically acceptable excipient and a pharmaceutically effective amount of a compound of the invention. A pharmaceutically acceptable excipient is any excipient which is relatively non-toxic and innocuous to a patient at concentrations consistent with effective activity of the active ingredient so that any side effects ascribable to the carrier do not vitiate the beneficial effects of the active ingredient. A pharmaceutically effective amount of compound is that amount which produces a result or exerts an influence on the particular condition being treated.

The term "the compound of formula (I)" or "refametinib" refers to (S)-N-(3,4-difluoro-2-(2-fluoro-4-iodophenylamino)-6-methoxyphenyl)-1-(2,3-dihydroxypropyl)cyclopropane-1-sulfonamide as depicted in formula (I).

The term "compound of the invention" or "active agent" or "active ingredient" refer to refametinib, a hydrate, solvate, metabolite or pharmaceutically acceptable salt of refametinib, or a polymorph thereof.

Solvates for the purposes of the invention are those forms of the compounds or their salts where solvent molecules form a stoichiometric complex in the solid state and include, but are not limited to for example water, ethanol and methanol.

Hydrates are a specific form of solvates, where the solvent molecule is water. Hydrates of the compounds of the invention or their salts are stoichiometric compositions of the compounds or salts with water, such as, for example, hemi-, mono- or dihydrates. Preference is given to the monohydrate of refametinib.

Salts for the purposes of the present invention are preferably pharmaceutically acceptable salts of the compounds according to the invention. Suitable pharmaceutically acceptable salts are well known to those skilled in the art and include salts of inorganic and organic acids, such as hydrochloric acid, hydrobromic acid, sulfuric acid, phosphoric acid, methanesulphonic acid, trifluoromethanesulfonic acid, benzenesulfonic acid, p-toluenesulfonic acid (tosylate salt), 1-naphthalenesulfonic acid, 2-naphthalenesulfonic acid, acetic acid, trifluoroacetic acid, malic acid, tartaric acid, citric acid, lactic acid, oxalic acid, succinic acid, fumaric acid, maleic acid, benzoic acid, salicylic acid, phenylacetic acid, and mandelic acid. In addition, pharmaceutically acceptable salts include salts of inorganic bases, such as salts containing alkaline cations (e.g., Li⁺ Na⁺ or K⁺), alkaline earth cations (e.g., Mg⁺² , Ca⁺² or Ba⁺²), the ammonium cation, as well as acid salts of organic bases, including aliphatic and aromatic substituted ammonium, and quaternary ammonium cations, such as those arising from protonation or peralkylation of triethylamine, *N,N*-diethylamine, *N,N*-dicyclohexylamine, lysine, pyridine, *N,N-*dimethylaminopyridine (DMAP), 1,4-diazabiclo[2.2.2]octane (DABCO), 1,5-diazabicyclo[4.3.0]non-5-ene (DBN) and 1,8-diazabicyclo[5.4.0]undec-7-ene (DBU). Preference is given to the hydrochloride, mesylate or phenylsulfonate salt of refametinib.

In accordance with an embodiment, the coated tablets of the present invention comprise refametinib which is substantially amorphous, such as refametinib as prepared and exemplified in WO 2007/014011 A2.

In accordance with an embodiment, the coated tablets of the present invention comprise refametinib which is substantially crystalline, such as refametinib prepared and exemplified in WO 2009/018233, and which is referred to herein as "polymorph A".

In accordance with an embodiment, the coated tablets of the present invention comprise refametinib which is crystalline.

In accordance with an embodiment, the coated tablets of the present invention comprise refametinib which is in the form of polymorph A as defined in WO 2009/018233, *supra.*

In accordance with an embodiment, the coated tablets of the present invention comprise refametinib which is used in crystalline form existing for at least 80% in the stable polymorph A as defined in WO 2009/018233, *supra.*

In accordance with an embodiment, the coated tablets of the present invention comprise refametinib which is crystalline, exists for at least 80% in the stable polymorph A as defined in WO 2009/018233 and is used in micronized form for manufacture of the pharmaceutical composition according to the present invention.

In accordance with an embodiment, the coated tablets of the present invention comprise refametinib which is in micronized form and which is in the form of polymorph A as defined in WO 2009/018233, *supra.*

Micronization can be achieved by standard milling methods, preferably by air jet milling, known to a skilled person. The mean particle size x50 of the micronized drug substance is preferably below 10 µm, more preferably in the range of 1 to 8 µm. The x90 is preferably max. 30 µm, more preferably max. 25 µm. The indicated particle sizes x50 (mean of the particle size distribution) and x90 (90% fraction) are measured by laser diffraction known to a skilled person (measuring device: HELOS, Sympatec).

Typical tablet stability-maintaining film formers are hydroxyethyl cellulose, hydroxypropyl cellulose, methyl cellulose, hydroxypropyl methyl cellulose, polyvinyl alcohol, polyvinylpyrrolidone, a copolymer of vinylpyrrolidone and vinylacetate sucrose, polyacrylate and polymethacrylate (such as copolymers of acryl- and/or methacrylic acid ester- with trimethylammoniummethylacrylat, copolymers of dimethylaminomethacrylic acid and neutral methacrylic acid esters, polymers of methacrylic acid or methacrylic acid esters, copolymer of acrylic acid ethyl ester and methacrylic acid methyl ester, copolymer of methacrylic acid and acrylic acid methyl ester), liquid glucose, ethyl cellulose, cellulose acetate phthalate, hydroxypropyl methyl cellulose acetate succinate, hydroxypropyl methyl cellulose phthalate and shellac. The coating agents can be mixed with further applicable coating excipients or commercially available ready-to-use coating mixtures can be used.

As mentioned *supra,* in accordance with a first aspect, the present invention as defined in the claims relates:
* to coated tablets comprising :
   - a core, comprising :
      ∘ refametinib, a hydrate, solvate, polymorph, metabolite thereof, or a pharmaceutically acceptable salt thereof; and
      ∘ one or more pharmaceutically acceptable core excipients; and
   - a polyethylene glycol-free, stability-maintaining coating, comprising :
      ∘ a stability-maintaining film former, which is hydroxypropyl methylcellulose ;
      ∘ optionally, a further stability-maintaining film former;
      ∘ and, optionally,
      ∘ one or more pharmaceutically acceptable coating excipients.

In accordance with an embodiment of the first aspect of the present invention, said coated tablets comprise said stability-maintaining film former which is selected from the group consisting of: hydroxyethyl cellulose, hydroxypropyl cellulose, methyl cellulose, hydroxypropyl methylcellulose (hypromellose (HMPC)), polyvinyl alcohol (PVA), polyvinylpyrrolidone, a copolymer of vinylpyrrolidone and vinylacetate (such as Kollidon^{®} VA64 BASF), a copolymer of vinylpyrrolidone and vinylacetate sucrose, polyacrylate and polymethacrylate (such as copolymers of acryl- and/or methacrylic acid ester- with trimethylammoniummethylacrylat, a copolymer of dimethylaminomethacrylic acid and a neutral methacrylic acid ester, a polymer of methacrylic acid or methacrylic acid esters, a copolymer of acrylic acid ethyl ester and methacrylic acid methyl ester, a copolymer of methacrylic acid and acrylic acid methyl ester, a copolymer of acrylic acid and acrylic acid methyl ester, liquid glucose, ethyl cellulose, cellulose acetate phthalate, hydroxypropyl methyl cellulose acetate succinate, hydroxypropyl methyl cellulose phthalate, and shellac, or a mixture thereof.

In accordance with an embodiment of the first aspect of the present invention, said refametinib is in micronized form.

In accordance with an embodiment of the first aspect of the present invention, said refametinib is in the form of polymorph A.

In accordance with an embodiment of the first aspect of the present invention, said further stability-maintaining film former is selected from the group consisting of: hydroxyethyl cellulose, hydroxypropyl cellulose, methyl cellulose, polyvinyl alcohol (PVA), polyvinylpyrrolidone, a copolymer of vinylpyrrolidone and vinylacetate (such as Kollidon^{®} VA64 BASF), a copolymer of vinylpyrrolidone and vinylacetate sucrose, polyacrylate and polymethacrylate (such as copolymers of acryl- and/or methacrylic acid ester- with trimethylammoniummethylacrylat, a copolymer of dimethylaminomethacrylic acid and a neutral methacrylic acid ester, a polymer of methacrylic acid or methacrylic acid esters, a copolymer of acrylic acid ethyl ester and methacrylic acid methyl ester, a copolymer of methacrylic acid and acrylic acid methyl ester, a copolymer of acrylic acid and acrylic acid methyl ester, liquid glucose, ethyl cellulose, cellulose acetate phthalate, hydroxypropyl methyl cellulose acetate succinate, hydroxypropyl methyl cellulose phthalate, and shellac, or a mixture thereof.

In accordance with an embodiment of the first aspect of the present invention, said further stability-maintaining film former is polyvinyl alcohol (PVA).

In accordance with an embodiment of the first aspect of the present invention, said hydroxypropyl methylcellulose is present in an amount of 30 to 100% by weight of said polyethylene glycol-free, stability-maintaining coating.

In accordance with an embodiment of the first aspect of the present invention, said hydroxypropyl methylcellulose is present in an amount of 40 to 55% by weight of said polyethylene glycol-free, stability-maintaining coating.

In accordance with an embodiment of the second aspect of the present invention, said coated tablets of the present invention comprise said stability-maintaining film former which is hydroxypropyl methylcellulose (hypromellose (HPMC)) and which is present in an amount of 50.6% by weight of said polyethylene glycol-free, stability-maintaining coating.

As mentioned *supra,* in accordance with a second aspect, the present invention as defined in the claims relates:
* to coated tablets comprising :
   - a core, comprising :
      ∘ refametinib, a hydrate, solvate, polymorph, metabolite thereof, or a pharmaceutically acceptable salt thereof; and
      ∘ one or more pharmaceutically acceptable core excipients; and
   - a stability-maintaining coating, comprising :
      ∘ a stability-maintaining film former, which is polyvinyl alcohol, particularly partially hydrolysed polyvinyl alcohol ;
      ∘ optionally, a further stability-maintaining film former;
      ∘ a plasticizer, which is polyethylene glycol ;
      ∘ and, optionally,
      ∘ one or more pharmaceutically acceptable coating excipients.

In accordance with an embodiment of the second aspect of the present invention, said refametinib is in micronized form.

In accordance with an embodiment of the second aspect of the present invention, said refametinib is in the form of polymorph A.

In accordance with an embodiment of the second aspect of the present invention, said further stability-maintaining film former is selected from the group consisting of: hydroxyethyl cellulose, hydroxypropyl cellulose, methyl cellulose, polyvinylpyrrolidone, a copolymer of vinylpyrrolidone and vinylacetate (such as Kollidon^{®} VA64 BASF), a copolymer of vinylpyrrolidone and vinylacetate sucrose, polyacrylate and polymethacrylate (such as copolymers of acryl- and/or methacrylic acid ester- with trimethylammoniummethylacrylat, a copolymer of dimethylaminomethacrylic acid and a neutral methacrylic acid ester, a polymer of methacrylic acid or methacrylic acid esters, a copolymer of acrylic acid ethyl ester and methacrylic acid methyl ester, a copolymer of methacrylic acid and acrylic acid methyl ester, a copolymer of acrylic acid and acrylic acid methyl ester, liquid glucose, ethyl cellulose, cellulose acetate phthalate, hydroxypropyl methyl cellulose acetate succinate, hydroxypropyl methyl cellulose phthalate, and shellac, or a mixture thereof.

In accordance with an embodiment of the second aspect of the present invention, said coated tablets comprise said stability-maintaining film former which is polyvinyl alcohol (partially hydrolysed) (PVA) and is present in an amount of 30 to 100% by weight of said stability-maintaining coating.

In accordance with an embodiment of the second aspect of the present invention, said coated tablets comprise said stability-maintaining film former which is polyvinyl alcohol (partially hydrolysed) (PVA) and is present in an amount of 30 to 60% by weight of said stability-maintaining coating.

In accordance with an embodiment of the second aspect of the present invention, said coated tablets comprise said stability-maintaining film former which is polyvinyl alcohol (partially hydrolysed) (PVA) and is present in an amount of 40 to 55% by weight of said stability-maintaining coating.

In accordance with an embodiment of the second aspect of the present invention, said coated tablets comprise said stability-maintaining film former which is polyvinyl alcohol (partially hydrolysed) (PVA) and is present in an amount of 44% by weight of said stability-maintaining coating.

In accordance with an embodiment of the first aspect of the present invention, said polyethylene glycol-free, stability-maintaining coating may be another HPMC-based coating system (comprising HPMC, talc and colorants (titanium dioxide and ferric oxide)).

In accordance with an embodiment of the second aspect of the present invention, said stability-maintaining coating may be an alternative PVA-based coating system (Opadry™ II 85G32407 yellow, comprising polyvinyl alcohol (partially hydrolyzed) [44% by weight of the total mixture], polyethylenglycol (PEG 3350) [12.4% by weight of the total mixture], lecithin (soya), ferric oxide, titanium dioxide and talc; or the lecithin-free variant thereof : the coated tablets of the present invention may also include a soy-free coating variant as defined herein, in particular a soy lecithin-free alternative of the Opadry coating, comprising only PVA, Macrogol (PEG) 3350,Talc, titanium dioxide and ferric oxides (red, yellow and/or black).

In accordance with an embodiment of the first or second aspect of the present invention, said one or more pharmaceutically acceptable coating excipients present in said stability-maintaining coating is (are) selected from the group consisting of :
* one or more plasticizers, which is not polyethylene glycol ;
* one or more colorants;
* one or more anti-tacking agents; and
* one or more opacifiers.

In accordance with an embodiment of the first or second aspect of the present invention, said one or more plasticizer(s) is (are) selected from the group consisting of : propylene glycol, polypropylene glycol, sorbitol, glycerol, maltitol, xylitol, mannitol, erythritol, glycerol trioleate, tributyl citrate, triethyl citrate, acetyl triethyl citrate, glyceryl triacetate, stearic acid, medium chain triglycerides, or a mixture thereof.

In accordance with an embodiment of the first or second aspect of the present invention, said one or more plasticizer(s) is (are) present in a total amount of 0 to 35% by weight of said stability-maintaining coating.

In accordance with an embodiment of the first or second aspect of the present invention, said one or more colorant(s) is (are) selected from the group consisting of: a natural or synthetic dye or pigment, such as ferric oxide red, ferric oxide yellow, ferric oxide black, titanium dioxide, indigotine, sunset yellow FCF, tartrazin, erythrosine, quinoline yellow, carbon black, anthocyanin, riboflavin, carmine, curcumin, chlorophyll, carotene, or a mixture thereof, preferably titanium dioxide and a ferric oxide, such as ferric oxide yellow, ferric oxide red and/or ferric oxide black, more preferably a ferric oxide and titanium dioxide.

In accordance with an embodiment of the first or second aspect of the present invention, said one or more colorant(s) is (are) present in a total amount of 10 to 50%, preferably 15 to 45%, by weight of said stability-maintaining coating.

In accordance with an embodiment of the first or second aspect of the present invention, said one or more anti-tacking agent(s) is (are) selected from the group consisting of : talc, magnesium stearate, stearic acid, lecithin, soy lecithin, mineral oil, carnauba wax, acetylated monoglycerides and/or polysorbate or a mixture thereof, preferably talc, lecithin, soy lecithin and/or polysorbate or a mixture thereof, more preferably talc, lecithin and soy lecithin.

In accordance with an embodiment of the first or second aspect of the present invention, said one or more anti-tacking agent(s) is (are) present in a total amount of 1 to 40%, preferably 3 to 25%, by weight of said stability-maintaining coating.

In accordance with an embodiment of the first or second aspect of the present invention, said one or more opacifier(s) is (are) selected from the group consisting of : talc and titanium dioxide.

In accordance with an embodiment of the first or second aspect of the present invention, said one or more opacifier(s) is (are) present in a total amount of 10 to 60% by weight of said stability-maintaining coating.

In accordance with an embodiment of the first or second aspect of the present invention, said one or more pharmaceutically acceptable core excipient(s) present in said core is (are) selected from the group consisting of :
* one or more fillers or dry binders ;
* one or more disintegrants ;
* one or more surfactants, particularly a wetting agent ;
* one or more binders (for wet granulation) ;
* one or more lubricants.

In accordance with an embodiment of the first or second aspect of the present invention, said filler or dry binder is selected from the group consisting of: powdered cellulose, microcrystalline cellulose, silicified microcrystalline cellulose, ethylcellulose, dicalcium phosphate, tricalcium phosphate, kaolin, magnesium trisilicate, mannitol, sorbitol, maltitol, xylitol, lactose (for example the anhydrous form or as hydrate, such as the monohydrate form), dextrose, maltose, sucrose, glucose, fructose or maltodextrines precipitated calcium carbonate, sodium carbonate, sodium phosphate and starch, preferably microcrystalline cellulose, mannitol, and/or lactose, more preferably mannitol, microcrystalline cellulose and/or lactose.

In accordance with an embodiment of the first or second aspect of the present invention, said filler or dry binder is present in an amount of 0 to 95%, preferably 30 to 80%, by weight of the total weight of said core.

In accordance with an embodiment of the first or second aspect of the present invention, said disintegrant is selected from the group consisting of: croscarmellose sodium, crospovidone (cross-linked polyvinylpyrrolidone), sodium starch glycollate, carboxymethylcellulose calcium, carboxymethylcellulose sodium, microcrystalline cellulose, hydroxypropyl cellulose, low substituted hydroxypropyl cellulose, polacrillin potassium, alginic acid, sodium alginate, partially hydrolysed starch, sodium carboxymethyl starch and starch, preferably croscarmellose sodium and/or crospovidone, more preferably croscarmellose sodium.

In accordance with an embodiment of the first or second aspect of the present invention, said disintegrant is present in an amount of 0 to 15%, preferably 3 to 10%, by weight of the total weight of said core.

In accordance with an embodiment of the first or second aspect of the present invention, said wetting agent (or "surfactant") is selected from the group consisting of: heptadecaethylene oxycetanol, lecithins, polyoxyethylene stearate, nonoxynol 9, nonoxynol 10, oxtoxynol 9, sorbitan fatty acid esters for example Span 20, 40, 60, 80 or 85, polysorbates for example polysorbate 20, 21, 40, 60, 61, 65 or 80, sodium salts of fatty alcohol sulfates such as sodium lauryl sulfate, sodium salts of sulfosuccinates such as sodium dioctylsulfosuccinate, partially esters of fatty acids with alcohols such as glycerine monostearate, ethers of fatty alcohols with polyoxyethylene, esters of fatty acids with polyoxyethylene, copolymers of ethylenoxide and propylenoxide (Pluronic^{®}), benzalkonium chloride and ethoxylated triglycerides, preferably sodium lauryl sulfate.

In accordance with an embodiment of the first or second aspect of the present invention, said wetting agent is present in an amount of 0 to 5%, preferably 0.1 to 2%, by weight of the total weight of said core.

In accordance with an embodiment of the first or second aspect of the present invention, said binder (for wet granulation) is selected from the group consisting of: hydroxypropyl cellulose, hypromellose (hydroxypropyl methylcellulose, HPMC), hydroxyethylcellulose, carboxymethylcellulose, carboxymethylcellulose calcium, carboxymethylcellulose sodium, ethylcellulose, methylcellulose, povidone (polyvinylpyrrolidone, PVP), polyvinyl alcohol, polyacrylates, gelatine, liquid glucose, arabic gum, alginic acid and pregelatinized starch, preferably a hydrophilic binder which is soluble in the aqueous granulation liquid, more preferably hypromellose (hydroxypropyl methylcellulose, HPMC) and/or polyvinylpyrrolidone, more preferably still hypromellose.

In accordance with an embodiment of the first or second aspect of the present invention, said binder, which is soluble in the aqueous granulation liquid, is present in an amount of 0 to 15%, preferably 0.5 to 8%, by weight of the total weight of said core.

In accordance with an embodiment of the first or second aspect of the present invention, said lubricant is selected from the group consisting of: calcium stearate, magnesium stearate, zinc stearate, stearic acid, fumaric acid, sodium stearylfumarate, glycerol dibehenate, glycerol distearate, glyceryl dipalmitostearate, behenoyl polyoxyl-8 glycerides mineral oil, and polyethylene glycol, preferably magnesium stearate.

In accordance with an embodiment of the first or second aspect of the present invention, said lubricant is present in an amount of 0 to 5%, preferably 0.2 to 3%, by weight of the total weight of said core.

In accordance with an embodiment of the first aspect of the present invention, said coated tablet comprises:
- a core, comprising :
   ∘ refametinib, which is micronized polymorph A; and
   ∘ pharmaceutically acceptable core excipients, which are :
      - a filler and dry binder, which is mannitol ;
      - a disintegrant, which is croscarmellose sodium ;
      - a surfactant, which is sodium lauryl sulfate ;
      - a binder, which is hypromellose ;
      - a lubricant, which is magnesium stearate;
      and
- a polyethylene glycol-free, stability-maintaining coating, comprising :
   ∘ a stability-maintaining film former, which is hydroxypropyl methylcellulose (hypromellose (HPMC));
   ∘ optionally a further stability-maintaining film former;
      and, optionally,
   ∘ pharmaceutically acceptable coating excipients, which are :
      - a colorant, which is a ferric oxide and/or titanium dioxide ;
      - an anti-tacking agent, which is talc, lecithin and/or soy lecithin ; and
      - an opacifier, which is talc and/or titanium dioxide.

In accordance with an embodiment of the first aspect of the present invention, said coated tablet comprises:
- a core, comprising :
   ∘refametinib, which is micronized polymorph A; and
   ∘ pharmaceutically acceptable core excipients, which are :
      - a filler and dry binder, which is mannitol ;
      - a disintegrant, which is croscarmellose sodium ;
      - a surfactant, which is sodium lauryl sulfate ;
      - a binder, which is hypromellose ;
      - a lubricant, which is magnesium stearate;
   and
- a stability-maintaining coating, comprising :
   ∘ a stability-maintaining film former, which is polyvinyl alcohol (PVA) ;
   ∘ polyethylene glycol (PEG), in an amount of 0 to 19% by weight of said stability-maintaining coating;
      and, optionally,
   ∘ pharmaceutically acceptable coating excipients, which are :
      - a colorant, which is a ferric oxide and/or titanium dioxide ;
      - an anti-tacking agent, which is talc, lecithin and/or soy lecithin ; and
      - an opacifier, which is talc and/or titanium dioxide.

In accordance with an embodiment of the first aspect of the present invention, said coated tablet comprises:
- a core, comprising :
   ∘ refametinib, which is micronized polymorph A; and
   ∘ pharmaceutically acceptable core excipients, which are :
      - a filler and dry binder, which is mannitol, and which is present in an amount of 0 to 95%, preferably 30 to 80%, by weight of the total weight of said core;
      - a disintegrant, which is crocarmellose sodium, contained in an amount of 0 to 15%, preferably 3 to 10%, by weight of the total weight of said core ;
      - a surfactant, which is sodium lauryl sulfate, which is present in an amount of 0.5 to 1%, preferably 0.1 to 2%, by weight of the total weight of said core;
      - a binder, which is hypromellose, which is present in an amount of 0 to 15%, preferably 0.5 to 8%, by weight of the total weight of said core ;
      - a lubricant, which is magnesium stearate, contained in an amount of 0 to 5%, preferably 0.2 to 3%, by weight of the total weight of said core ;
   and
- a polyethylene glycol-free, stability-maintaining coating, comprising :
   ∘ a stability-maintaining film former, which is :
      ▪ hydroxypropyl methylcellulose (hypromellose (HPMC)), present in an amount of 30 to 100%, preferably 40 to 55%, by weight of said polyethylene glycol-free, stability-maintaining coating;
         and, optionally,
   ∘ pharmaceutically acceptable coating excipients, which are :
      - a colorant, which is a ferric oxide or titanium dioxide, present in an amount of 10 to 50% by weight of said polyethylene glycol-free, stability-maintaining coating;
      - an anti-tacking agent, which is talc, present in an amount of 1 to 40%, preferably 3 to 25%, by weight of said polyethylene glycol-free, stability-maintaining coating; and
      - an opacifier, which is talc and/or titanium dioxide, present in an amount of 10 to 60% by weight of said polyethylene glycol-free, stability-maintaining coating.

In accordance with an embodiment of the second aspect of the present invention, said coated tablet comprises:
- a core, comprising :
   ∘ refametinib, which is micronized polymorph A; and
   ∘ pharmaceutically acceptable core excipients, which are :
      - a filler and dry binder, which is mannitol, and which is present in an amount of 0 to 95%, preferably 30 to 80%, by weight of the total weight of said core;
      - a disintegrant, which is crocarmellose sodium, contained in an amount of 0 to 15%, preferably 3 to 10%, by weight of the total weight of said core ;
      - a surfactant, which is sodium lauryl sulfate, which is present in an amount of 0.5 to 1%, preferably 0.1 to 2%, by weight of the total weight of said core;
      - a binder, which is hypromellose, which is present in an amount of 0 to 15%, preferably 0.5 to 8%, by weight of the total weight of said core ;
      - a lubricant, which is magnesium stearate, contained in an amount of 0 to 5%, preferably 0.2 to 3%, by weight of the total weight of said core ;
   and
- a stability-maintaining coating, comprising :
   ∘ a stability-maintaining film former, which is :
      ▪ polyvinyl alcohol (partially hydrolysed) (PVA), present in an amount of 30 to 100%, preferably 40 to 55%, by weight of said stability-maintaining coating;
      ▪ polyethylene glycol (PEG), present in an amount of 0 to 19% by weight of said stability-maintaining coating;
         and, optionally,
   ∘ pharmaceutically acceptable coating excipients, which are :
      - a colorant, which is a ferric oxide or titanium dioxide, present in an amount of 10 to 50% by weight of said stability-maintaining coating;
      - an anti-tacking agent, which is talc, present in an amount of 1 to 40%, preferably 3 to 25%, by weight of said stability-maintaining coating; and
      - an opacifier, which is talc and/or titanium dioxide, present in an amount of 10 to 60% by weight of said stability-maintaining coating.

In accordance with an embodiment of the first or second aspect of the present invention, said coated tablets contain 10, 20, 30 or 50 mg refametinib.

In accordance with an embodiment of the first or second aspect of the present invention, said coated tablets of the present invention contain 10 mg refametinib.

In accordance with an embodiment of the first or second aspect of the present invention, said coated tablets of the present invention contain 20 mg refametinib.

In accordance with an embodiment of the first or second aspect of the present invention, said coated tablets of the present invention contain 30 mg refametinib.

In accordance with an embodiment of the first or second aspect of the present invention, said coated tablets of the present invention contain 50 mg refametinib.

The total amount of the active ingredient (refametinib) to be administered preferably via the oral route using the pharmaceutical composition of the present invention will generally range from about 0.1 mg/kg to about 50 mg/kg body weight per day. Based upon standard laboratory techniques known to evaluate compounds useful for the treatment of hyper-proliferative disorders, by standard toxicity tests and by standard pharmacological assays for the determination of treatment of the conditions identified above in mammals, and by comparison of these results with the results of known medicaments that are used to treat these conditions, the effective dosage of the pharmaceutical compositions of this invention can readily be determined by those skilled in the art. The amount of the administered active ingredient can vary widely according to such considerations as the particular compound and dosage unit employed, the mode and time of administration, the period of treatment, the age, sex, and general condition of the patient treated, the nature and extent of the condition treated, the rate of drug metabolism and excretion, the potential drug combinations and drug-drug interactions, and the like.

Preference is given to an amount of refametinib in the pharmaceutical composition from 4 to 400 mg, preferably from 10 to 200 mg, more preferably from 10 to 100 mg, in particular 10, 20, 30 or 50 mg.

An aspect of the invention of particular interest is a pharmaceutical composition comprising refametinib in an amount of 4 to 400 mg, preferably from 10 to 200 mg, more preferably from 10 to 100 mg, in particular 10, 20, 30 or 50 mg.

The daily dose of refametinib is from 4 to 800 mg, preferably from 10 to 400 mg, more preferably from 10 to 200 mg, in particular 20, 40, 60 or 100 mg.

The pharmaceutical composition according to the invention is administered one or more, preferably up to three, more preferably up to two times per day. Preference is given to an administration via the oral route. With each administration the number of tablets or capsules taken in at the same time preferably does not exceed two.

Nevertheless, it may in some cases be advantageous to deviate from the amounts specified, depending on body weight, sex and/or age and general condition of the patient, individual behaviour toward the active ingredient, type of preparation and time or interval over which the administration is affected. For instance, less than the aforementioned minimum amounts may be sufficient in some cases, while the upper limit specified has to be exceeded in other cases. In the case of administration of relatively large amounts, it may be advisable to divide these into several individual doses over the day.

This pharmaceutical composition will be utilized to achieve the desired pharmacological effect by preferably oral administration to a patient in need thereof, and will have advantageous properties in terms of drug release, bioavailability, and/or compliance in mammals. A patient, for the purpose of this invention, is a mammal, including a human, in need of treatment for the particular condition or disease.

Preference is given to a pharmaceutical composition which is an immediate release tablet.

The pharmaceutical composition according to the invention is preferably a solid pharmaceutical composition and is administered orally or rectally, preferably orally.

The tablet core of the pharmaceutical composition of the present invention comprises optionally one or more pharmaceutical acceptable excipients such fillers and dry binders, binders, disintegrants, surfactants and wetting agents, suspending agents, glidants and lubricants.

Fillers and dry binders include, but are not limited to powdered cellulose, microcrystalline cellulose, silicified microcrystalline cellulose, ethylcellulose, dicalcium phosphate, tricalcium phosphate, kaolin, magnesium trisilicate, mannitol, sorbitol, maltitol, xylitol, lactose (for example the anhydrous form or as hydrate, such as the monohydrate form), dextrose, maltose, sucrose, glucose, fructose or maltodextrines precipitated calcium carbonate, sodium carbonate, sodium phosphate and starch. Preference is given to microcrystalline cellulose, mannitol, and/or lactose, more preference is given mannitol.

Disintegrants include, but are not limited to croscarmellose sodium, crospovidone (cross-linked polyvinylpyrrolidone), sodium starch glycollate, carboxymethylcellulose calcium, carboxymethylcellulose sodium, microcrystalline cellulose, hydroxypropyl cellulose, low substituted hydroxypropyl cellulose, polacrillin potassium, alginic acid, sodium alginate, partially hydrolysed starch, sodium carboxymethyl starch and starch. Preference is given to croscarmellose sodium and/or crospovidone, more preference is given to croscarmellose sodium.

Binders (for wet granulation) include, but are not limited to hydroxypropyl cellulose, hypromellose (hydroxypropyl methylcellulose, HPMC), hydroxyethylcellulose, carboxymethylcellulose, carboxymethylcellulose calcium, carboxymethylcellulose sodium, ethylcellulose, methylcellulose, povidone (polyvinylpyrrolidone, PVP), polyvinyl alcohol, polyacrylates, gelatine, liquid glucose, arabic gum, alginic acid and pregelatinized starch. Preference is given to a hydrophilic binder which is soluble in the aqueous granulation liquid, more preference is given to hypromellose (hydroxypropyl methylcellulose, HPMC) and/or polyvinylpyrrolidone, most preference is given to hypromellose.

Surfactants and wetting agents include, but are not limited to heptadecaethylene oxycetanol, lecithins, polyoxyethylene stearate, nonoxynol 9, nonoxynol 10, oxtoxynol 9, sorbitan fatty acid esters for example Span 20, 40, 60, 80 or 85, polysorbates for example polysorbate 20, 21, 40, 60, 61, 65 or 80, sodium salts of fatty alcohol sulfates such as sodium lauryl sulfate, sodium salts of sulfosuccinates such as sodium dioctylsulfosuccinate, partially esters of fatty acids with alcohols such as glycerine monostearate, ethers of fatty alcohols with polyoxyethylene, esters of fatty acids with polyoxyethylene, copolymers of ethylenoxide and propylenoxide (Pluronic^{®}), benzalkonium chloride and ethoxylated triglycerides. Preference is given to sodium lauryl sulfate.

Lubricants include, but are not limited to calcium stearate, magnesium stearate, zinc stearate, stearic acid, fumaric acid, sodium stearylfumarate, glycerol dibehenate, glycerol distearate glyceryl dipalmitostearate, behenoyl polyoxyl-8 glycerides mineral oil, , and polyethylene glycol. Preference is given to magnesium stearate.

Furthermore the coating of the pharmaceutical composition of the present invention comprises optionally one or more further pharmaceutically acceptable excipients such as plasticizers, colorants, opacifiers, anti-tacking agents, dispersing agents and suspending agents.

All publications, applications and patents cited above and below are incorporated herein by reference.

The weight data are, unless stated otherwise, percentages by weight and parts are parts by weight.

### EXAMPLES

### Example 1: Coated tablet comprising refametinib

### a) Granulation

For manufacture of 20 mg and 30 mg tablets, a solution of 0.050 kg hypromellose 5 cP and 0.016 kg sodium laurylsulfate in 2.30 kg water was prepared. Subsequently 0.417 kg refametinib micronized was added to this solution and suspended homogeneously. Using a fluidized bed granulator the resulting suspension was sprayed onto a powder bed 1.79 kg mannitol and 0.125 kg croscarmellose sodium at a product temperature of 28-34°C.

For manufacture of 50 mg tablets a solution of 0.050 kg hypromellose 5 cP and 0.016 kg sodium laurylsulfate in 2.30 kg water was prepared. Subsequently 0.500 kg refametinib micronized was added to this solution and suspended homogeneously. Using a fluidized bed granulator the resulting suspension was sprayed onto a powder bed 1.70 kg mannitol and 0.125 kg croscarmellose sodium at a product temperature of 28-34°C.

### b) Tableting

The granulates of step a) were screened 0.8 mm. Subsequently the granulates were blended with 0.075 kg croscarmellose sodium and 0.031 kg magnesium stearate. These ready -to-press blends were compressed on a rotary tablet press into tablets containing 20 mg, 30 mg and 50 mg of refamentinib, respectively.

### c) Film coating

For coating of the 20 mg tablets 52.7 g Hypromellose 5 cP, 10.6 g talc, 38.8 g titianium dioxide and 2.1 g ferric oxide yellow were homogeneously dispersed in 0.590 kg water.

For coating of the 30 mg tablets 42.1 g Hypromellose 5 cP, 8.5 g talc, 22.9 g titianium dioxide and 9.9 g ferric oxide yellow were homogeneously dispersed in 0.470 kg water.

For coating of the 50 mg tablets 40.5 g Hypromellose 5 cP, 8.1 g talc and 31.4 g ferric oxide yellow were homogeneously dispersed in 0.450 kg water.

These coating suspensions were sprayed onto the 20 mg, 30 mg respectively 50 mg tablets of step b) in a perforated drum coater at an outlet air temperature of 40-55°C. The coating process resulted in evenly coated tablets with a smooth surface. Coating defects could not be observed.

**Table 1: Composition of tablets of Example 1 containing refametinib**

| | Tablet 20 mg | Tablet 30 mg | Tablet 50 mg |
|---|---|---|---|
| | [mg/tablet] | [mg/tablet] | [mg/tablet] |
| CORE | | | |
| Refametinib micronized | 20.00 | 30.00 | 50.00 |
| Mannitol | 85.73 | 128.60 | 170.32 |
| Croscarmellose sodium | 9.60 | 14.40 | 20.00 |
| Natrium laurylsulfate | 0.77 | 1.15 | 1.55 |
| Hypromellose 5 cP | 2.40 | 3.60 | 5.00 |
| Magnesium stearate | 1.50 | 2.25 | 3.13 |

| COATING | | | |
|---|---|---|---|
| Hypromellose 5 cP | 2.53 | 3.03 | 4.05 |
| Talc | 0.51 | 0.61 | 0.81 |
| Ferric oxide yellow | 0.10 | 0.71 | 3.14 |
| Titanium oxide | 1.86 | 1.65 | - |
| Sum | 125.00 | 186.00 | 258.00 |
| Tablet format | Round | Round | round |
| Dimensions of the tablet | diameter: 7 mm | diameter: 8 mm | diameter: 9 mm |

### Example 2: Coated tablet comprising refametinib

### a) Granulation

For manufacture of 20 mg and 30 mg tablets, a solution of 0.089 kg hypromellose 5 cP and 0.016 kg sodium laurylsulfate in 1.55 kg water was prepared. Using a fluidized bed granulator this solution was sprayed onto a powder bed of 0.400 kg refametinib micronized, 1.66 kg mannitol and 0.120 kg croscarmellose sodium at a product temperature of 28-34°C.

For manufacture of 50 mg tablets a solution of 0.089 kg hypromellose 5 cP and 0.016 kg sodium laurylsulfate in 1.55 kg water was prepared. Using a fluidized bed granulator this solution was sprayed onto a powder bed of 0.485 kg refametinib micronized, 1.59 kg mannitol and 0.121 kg croscarmellose sodium at a product temperature of 28-34°C.

### b) Tableting

The granulates of step a) were screened 0.8 mm. Subsequently the granulate intended for manufacture of 20 mg or 30 mg tablets was blended with 0.072 kg croscarmellose sodium and 0.048 kg magnesium stearate. This ready -to-press blend was compressed on a rotary tablet press into tablets containing 20 mg and 30 mg of refamentinib. The granulate for the 50 mg tablets was blended with 0.073 kg croscarmellose sodium and 0.049 kg magnesium stearate. This ready -to-press blend was compressed on a rotary tablet press into tablets containing 50 mg of refamentinib.

### c) Film coating

For coating of the 20 mg tablets 50.6 g Hypromellose 5 cP, 10.2 g talc, 37.2 g titianium dioxide and 2.0 g ferric oxide yellow were homogeneously dispersed in 0.570 kg water.

For coating of the 30 mg tablets 40.7 g Hypromellose 5 cP, 8.2 g talc, 22.2 g titianium dioxide and 9.5 g ferric oxide yellow were homogeneously dispersed in 0.460 kg water.

For coating of the 50 mg tablets 39.2 g Hypromellose 5 cP, 7.8 g talc and 30.4 g ferric oxide yellow were homogeneously dispersed in 0.440 kg water.

These coating suspensions were sprayed onto the 20 mg, 30 mg respectively 50 mg tablets of step b) in a perforated drum coater at an outlet air temperature of 40-55°C. The coating process resulted in evenly coated tablets with a smooth surface. Coating defects could not be observed.

**Table 2: Composition of tablets of example 2 containing refametinib**

| | **Tablet 20 mg** | **Tablet 30 mg** | **Tablet 50 mg** |
|---|---|---|---|
| | [mg/tablet] | [mg/tablet] | [mg/tablet] |
| CORE | | | |
| Refametinib micronized | 20.00 | 30.00 | 50.00 |
| Mannitol | 82.80 | 124.20 | 164.20 |
| Croscarmellose sodium | 9.60 | 14.40 | 20.00 |
| Sodium laurylsulfate | 0.77 | 1.16 | 1.60 |
| Hypromellose 5 cP | 4.43 | 6.64 | 9.20 |
| Magnesium stearate | 2.40 | 3.60 | 5.00 |

| COATING | | | |
|---|---|---|---|
| Hypromellose 5 cP | 2.53 | 3.03 | 4.05 |
| Talc | 0.51 | 0.61 | 0.81 |
| Ferric oxide yellow | 0.10 | 0.71 | 3.14 |
| Titanium oxide | 1.86 | 1.65 | - |
| Sum | 125.00 | 186.00 | 258.00 |
| Tablet format | Round | Round | round |
| Dimensions of the tablet | diameter: 7 mm | diameter: 8 mm | diameter: 9 mm |

### Example 3: Coated tablet comprising refametinib

### a) Granulation

For manufacture of 20 mg and 30 mg tablets, a solution of 0.096 kg hypromellose 5 cP and 0.016 kg sodium laurylsulfate in 2.30 kg water was prepared. Subsequently 0.417 kg refametinib micronized was added to this solution and suspended homogeneously. Using a fluidized bed granulator the resulting suspension was sprayed onto a powder bed 1.84 kg mannitol at a product temperature of 28-34°C.

For manufacture of 50 mg tablets a solution of 0.092 kg hypromellose 5 cP and 0.016 kg sodium laurylsulfate in 2.30 kg water was prepared. Subsequently 0.500 kg refametinib micronized was added to this solution and suspended homogeneously. Using a fluidized bed granulator the resulting suspension was sprayed onto a powder bed 1.77 kg mannitol at a product temperature of 28-34°C.

### b) Tableting

The granulates of step a) were screened 0.8 mm. Subsequently the granulates for the 20 mg and 30 mg tablets were blended with 0.096 kg croscarmellose sodium and 0.031 kg magnesium stearate. The granulate for the 50 mg tablets was blended with 0.092 kg croscarmellose sodium and 0.031 kg magnesium stearate. The resulting ready -to-press blends were compressed on a rotary tablet press into tablets containing 20 mg, 30 mg and 50 mg of refamentinib, respectively.

### c) Film coating

For coating of the 20 mg tablets 104.2 g Opadry™ II 85G32407 yellow were homogeneously dispersed in 0.590 kg water.

For coating of the 30 mg tablets 83.4 g Opadry™ II 85G32407 yellow were homogeneously dispersed in 0.470 kg water.

For coating of the 50 mg tablets 80.0 g Opadry™ II 85G32407 yellow were homogeneously dispersed in 0.450 kg water.

These coating suspensions were sprayed onto the 20 mg, 30 mg respectively 50 mg tablets of step b) in a perforated drum coater at an outlet air temperature of 35-45°C. The coating process resulted in evenly coated tablets with a smooth surface. Coating defects could not be observed.

Commercially available Opadry™ II 85G32407 yellow contains polyvinyl alcohol (partially hydrolyzed) [44% by weight of the total mixture], polyethylenglycol (PEG 3350) [12.4% by weight of the total mixture], lecithin (soya), ferric oxide, titanium dioxide and talc.

**Table 3: Composition of tablets of example 3 containing refametinib**

| | Tablet 20 mg | Tablet 30 mg | Tablet 50 mg |
|---|---|---|---|
| | [mg/tablet] | [mg/tablet] | [mg/tablet] |
| CORE | | | |
| Refametinib micronized | 20.00 | 30.00 | 50.00 |
| Mannitol | 88.51 | 132.78 | 176.86 |
| Croscarmellose sodium | 4.61 | 6.91 | 9.23 |
| Sodium laurylsulfate | 0.77 | 1.15 | 1.55 |
| Hypromellose 5 cP | 4.61 | 6.91 | 9.23 |
| Magnesiumstearat | 1.50 | 2.25 | 3.13 |

| COATING | | | |
|---|---|---|---|
| Opadry II 85G32407 yellow | 5.00 | 6.00 | 8.00 |
| Sum | 125.00 | 186.00 | 258.00 |
| Tablet format | Round | round | round |
| Dimensions of the tablet | diameter: 7 mm | diameter: 8 mm | diameter: 9 mm |

### Comparative Example A: Tablet coated with a standard HPMC coating for comparison

Tablet cores equivalent to the uncoated tablets manufactured as described in Example 3 (a-b) were manufactured and film-coated as follows:
For coating of the 20 mg tablets 62.5 g Hypromellose 15 cP, 20.8 g PEG 3350, 19.8 g titianium dioxide and 1.0 g ferric oxide yellow were homogeneously dispersed in 1.28 kg water.

For coating of the 30 mg tablets 50.0 g Hypromellose 15 cP, 16.7 g PEG 3350, 11.7 g titianium dioxide and 5.0 g ferric oxide yellow were homogeneously dispersed in 1.03 kg water.

For coating of the 50 mg tablets 48.0 g Hypromellose 15 cP, 16.0 g PEG 3350 and 16.0 g ferric oxide yellow were homogeneously dispersed in 0.98 kg water.

These coating suspensions were sprayed onto the 20 mg, 30 mg respectively 50 mg tablet cores in a perforated drum coater at an outlet air temperature of 40-55°C. The coating process resulted in evenly coated tablets with a smooth surface. Coating defects could not be observed.

**Table 4: Composition of tablets of comparative example A containing refametinib**

| | Tablet 20 mg | Tablet 30 mg | Tablet 50 mg |
|---|---|---|---|
| | [mg/tablet] | [mg/tablet] | [mg/tablet] |
| CORE | | | |
| Refametinib micronized | 20.00 | 30.00 | 50.00 |
| Mannitol | 88.51 | 132.78 | 176.86 |
| Croscarmellose sodium | 4.61 | 6.91 | 9.23 |
| Sodium laurylsulfate | 0.77 | 1.15 | 1.55 |
| Hypromellose 5 cP | 4.61 | 6.91 | 9.23 |
| Magnesiumstearat | 1.50 | 2.25 | 3.13 |

| COATING | | | |
|---|---|---|---|
| Hypromellose 15 cP | 3.00 | 3.60 | 4.80 |
| PEG 3350 | 1.00 | 1.20 | 1.60 |
| Ferric oxide yellow | 0.05 | 0.36 | 1.60 |
| Titanium oxide | 0.95 | 0.84 | - |
| Sum | 125.00 | 186.00 | 258.00 |
| Tablet format | Round | Round | Round |
| Dimensions of the tablet | diameter: 7 mm | diameter: 8 mm | diameter: 9 mm |

### Test results:

### Comparison between Examples 1 to 3 and Comparative Example A

In the following a comparison of the dissolution results of the 30 mg dose strength of the formulations of Example 1-3 and Example A is presented exemplarily. However, the conclusions drawn are valid also for the other dose strengths, as comparable results were found also for the 20 mg and 50 mg tablets.

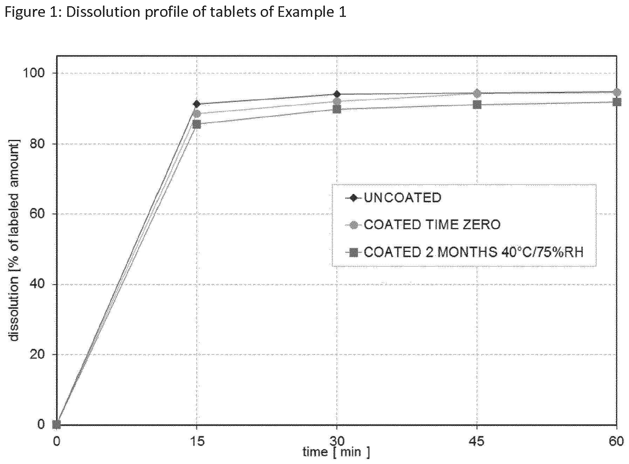

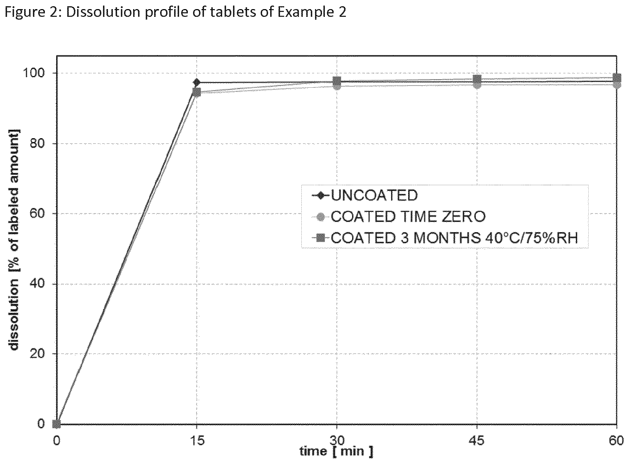

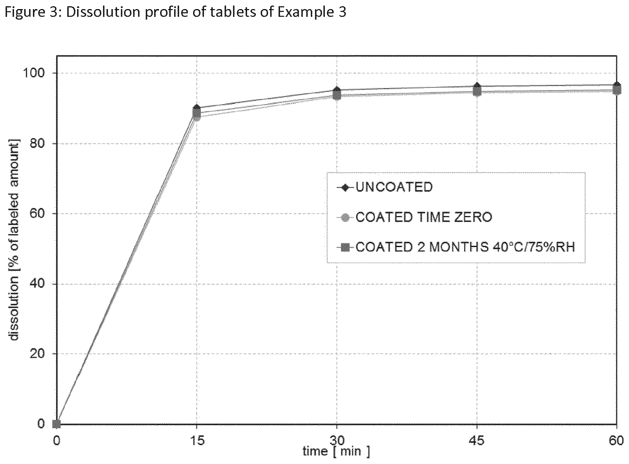

Dissolution testing (paddle apparatus, 900 mL 0.1 M HCL with 0.05% sodium laurylsulfate, 75 rpm, 37°C) was performed with the uncoated tablet cores and the coated tablets of Examples 1-3 and Examples A immediately after manufacture. In addition the coated tablets were stored under stress conditions (40°C/75% relative humidity) in a moisture permeable standard packaging material (HDPE bottles) for a storage period of at least 2 months. After storage under elevated temperature and humidity the coated tablets were subjected again to the dissolution test (Fig. 1-4).

For all the examples given, dissolution of the tablets was not influenced significantly by application of a coating regardless of the type of coating applied.

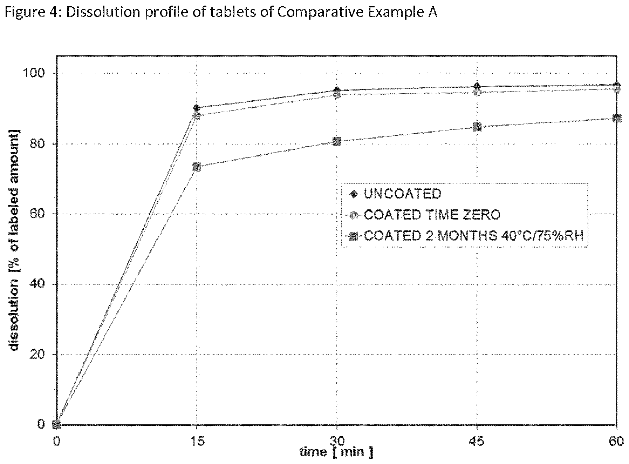

Storage under stress conditions, however, led to a significant impairment of the dissolution of the tablets of Comparative Example A coated with a standard HPMC coating (Fig 4). By storage at 40°C/75% relative humidity for 2 months, the amount of drug released after 15 minutes, 30 minutes and 60 minutes was reduced by 14.5%, 13.2% and 8.5%, respectively, indicating severe storage instability for the coated tablets of Comparative Example A.

Applying a different coating system according to Examples 1, 2 and 3 of the current invention on the same or a comparable tablet core, a stable formulation was achieved. Dissolution from coated tablets of Examples 1, 2 and 3 was not adversely affected by storage at 40°C/75% relative humidity for 2 or 3 months respectively (Fig. 1-3).

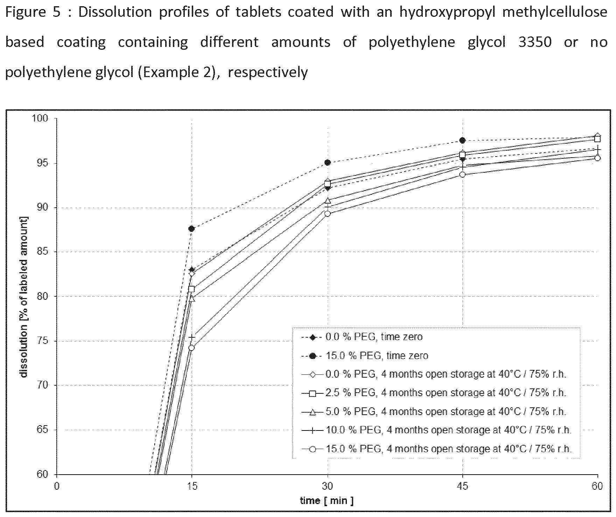

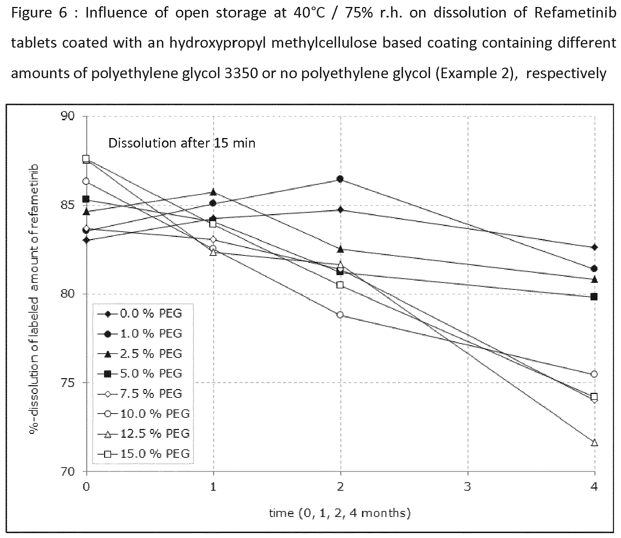

Surprisingly, significant amounts of the plasticizer polyethylene glycol 3350 in the coating, as present in many standard hydroxypropyl methylcellulose based coatings, impair the stability of Refametinib immediate release tablets significantly. After storage under humidity stress conditions (open storage at 40°C, 75% relative humidity (r.h)) tablets containing significant amounts of polyethylene glycol 3350 show clearly slower and less complete drug release: Dissolution in the initial phase is significantly slower (Fig. 5 and 6) and the plateau of the dissolution reached after 60 min is significantly lower compared to Refametinib tablets of Example 2 with the polyethylene glycol-free coating (Fig 5).

Hence, in accordance with an embodiment, the present invention also relates to a method of preparing a coated tablet as defined herein, comprising the following steps :
a) granulation, in which :
   i) said binder and said wetting agent are placed in water and dissolved, thus providing a granulation liquid ;
   ii) said granulation liquid is applied, such as by spraying for example, onto a mixture of micronized polymorph A refametinib, said filler/dry binder and said disintegrant, as a powder bed for example, in a fluidized bed granulator for example, at a temperature such as of 20 to 35°C for example, preferably of 28 to 34°C ;
   thus providing a refametinib-containing granulate ;
b) tableting, in which :
   i) said granulate is screened to a suitable size, such as 0.5 to 1.0 mm for example, preferably 0.8 mm, thus providing a screened refametinib-containing granulate ;
   ii) blending said screened refametinib-containing granulate with said disintegrant and said lubricant, thus providing a ready-to-press refametinib-containing blend ;
   iii) compressing said refametinib-containing blend into tablets, such as on a rotary tablet press for example, thus providing refametinib-containing uncoated tablets, containing 10, 20, 30 or 50 mg refametinib, for example ;
c) film coating, in which :
   i) dispersing said stability-maintaining film former, said anti-tacking agent, said opacifier and said colorants as well as optionally said plasticizers in water, thus providing a coating dispersion ;
   ii) applying, such as by spraying for example, said coating dispersion onto said refametinib-containing uncoated tablets, such as in a perforated drum coater for example, at a temperature of 35 to 60°C for example, preferably of 40 to 55°C ;
   thus providing coated tablets of the present invention as defined herein.

In accordance with an embodiment, the present invention relates to a method of preparing a coated tablet as defined herein, comprising the following steps :
a) granulation, in which :
   i) said binder and said wetting agent are placed in water and dissolved to form a solution in which said refametinib is suspended, thus providing a refametinib-containing granulation suspension;
   ii) said refametinib-containing granulation suspension is applied, such as by spraying for example, to said a mixture of filler/dry binder and said disintegrant, such as in a powder bed for example, in a fluidized bed granulator for example, at a temperature such as of 20 to 35°C for example, preferably of 28 to 34°C ;
   thus providing a refametinib-containing granulate ;
b) tableting, in which :
   i) said granulate is screened to a suitable size, such as 0.5 to 1.0 mm for example, preferably 0.8 mm, thus providing a screened refametinib-containing granulate ;
   ii) blending said screened refametinib-containing granulate with said disintegrant and said lubricant, thus providing a ready-to-press refametinib-containing blend;
   iii) compressing said refametinib-containing blend into tablets, such as on a rotary tablet press for example, thus providing refametinib-containing uncoated tablets, containing 10, 20, 30 or 50 mg refametinib, for example ;
c) film coating, in which :
   i) dispersing said stability-maintaining film former, said anti-tacking agent, said opacifiers and said colorants in water as well as optionally said plasticizers, thus providing a coating dispersion ;
   ii) applying, such as by spraying for example, said coating dispersion onto said refametinib-containing uncoated tablets, such as in a perforated drum coater for example, at a temperature of 35 to 60°C for example, preferably of 40 to 55°C ;
   thus providing coated tablets of the present invention as defined herein.

### Combination therapies

The coated tablets of this invention can be administered as the sole pharmaceutical agent or in combination with one or more other pharmaceutical agents where the combination causes no unacceptable adverse effects. The present invention relates also to such combinations.

"Combination" means for the purposes of the invention not only a dosage form which contains all the components (so-called fixed combinations), and combination packs containing the components separate from one another, but also components which are administered simultaneously or sequentially, as long as they are employed for the prophylaxis or treatment of the same disease.

For example, the coated tablets of this invention can be combined with known anti-hyper-proliferative or other indication agents, and the like, as well as with admixtures and combinations thereof. Other indication agents include, but are not limited to, anti-angiogenic agents, mitotic inhibitors, alkylating agents, anti-metabolites, DNA-intercalating antibiotics, growth factor inhibitors, cell cycle inhibitors, enzyme inhibitors, toposisomerase inhibitors, biological response modifiers, or anti-hormones.

In accordance with an embodiment, the present invention relates to pharmaceutical combinations comprising :
- a coated tablet of the present invention as defined herein, and
- one or more second active ingredients selected from chemotherapeutic anti-cancer agents.

The term "chemotherapeutic anti-cancer agents", includes but is not limited to :
131I-chTNT, abarelix, abiraterone, aclarubicin, aldesleukin, alemtuzumab, alitretinoin, altretamine, aminoglutethimide, amrubicin, amsacrine, anastrozole, arglabin, arsenic trioxide, asparaginase, azacitidine, basiliximab, copanlisib (BAY 80-6946), BAY 1000394, belotecan, bendamustine, bevacizumab, bexarotene, bicalutamide, bisantrene, bleomycin, bortezomib, buserelin, busulfan, cabazitaxel, calcium folinate, calcium levofolinate, capecitabine, carboplatin, carmofur, carmustine, catumaxomab, celecoxib, celmoleukin, cetuximab, chlorambucil, chlormadinone, chlormethine, cisplatin, cladribine, clodronic acid, clofarabine, crisantaspase, cyclophosphamide, cyproterone, cytarabine, dacarbazine, dactinomycin, darbepoetin alfa, dasatinib, daunorubicin, decitabine, degarelix, denileukin diftitox, denosumab, deslorelin, dibrospidium chloride, docetaxel, doxifluridine, doxorubicin, doxorubicin + estrone, eculizumab, edrecolomab, elliptinium acetate, eltrombopag, endostatin, enocitabine, epirubicin, epitiostanol, epoetin alfa, epoetin beta, eptaplatin, eribulin, erlotinib, estradiol, estramustine, etoposide, everolimus, exemestane, fadrozole, filgrastim, fludarabine, fluorouracil, flutamide, formestane, fotemustine, fulvestrant, gallium nitrate, ganirelix, gefitinib, gemcitabine, gemtuzumab, glutoxim, goserelin, histamine dihydrochloride, histrelin, hydroxycarbamide, I-125 seeds, ibandronic acid, ibritumomab tiuxetan, idarubicin, ifosfamide, imatinib, imiquimod, improsulfan, interferon alfa, interferon beta, interferon gamma, ipilimumab, irinotecan, ixabepilone, lanreotide, lapatinib, lenalidomide, lenograstim, lentinan, letrozole, leuprorelin, levamisole, lisuride, lobaplatin, lomustine, lonidamine, masoprocol, medroxyprogesterone, megestrol, melphalan, mepitiostane, mercaptopurine, methotrexate, methoxsalen, Methyl aminolevulinate, methyltestosterone, mifamurtide, miltefosine, miriplatin, mitobronitol, mitoguazone, mitolactol, mitomycin, mitotane, mitoxantrone, nedaplatin, nelarabine, nilotinib, nilutamide, nimotuzumab, nimustine, nitracrine, ofatumumab, omeprazole, oprelvekin, oxaliplatin, p53 gene therapy, paclitaxel, palifermin, palladium-103 seed, pamidronic acid, panitumumab, pazopanib, pegaspargase, PEG-epoetin beta (methoxy PEG-epoetin beta), pegfilgrastim, peginterferon alfa-2b, pemetrexed, pentazocine, pentostatin, peplomycin, perfosfamide, picibanil, pirarubicin, plerixafor, plicamycin, poliglusam, polyestradiol phosphate, polysaccharide-K, porfimer sodium, pralatrexate, prednimustine, procarbazine, quinagolide, raloxifene, raltitrexed, ranimustine, razoxane, regorafenib, risedronic acid, rituximab, romidepsin, romiplostim, sargramostim, sipuleucel-T, sizofiran, sobuzoxane, sodium glycididazole, sorafenib, streptozocin, sunitinib, talaporfin, tamibarotene, tamoxifen, tasonermin, teceleukin, tegafur, tegafur + gimeracil + oteracil, temoporfin, temozolomide, temsirolimus, teniposide, testosterone, tetrofosmin, thalidomide, thiotepa, thymalfasin, tioguanine, tocilizumab, topotecan, toremifene, tositumomab, trabectedin, trastuzumab, treosulfan, tretinoin, trilostane, triptorelin, trofosfamide, tryptophan, ubenimex, valrubicin, vandetanib, vapreotide, vemurafenib, vinblastine, vincristine, vindesine, vinflunine, vinorelbine, vorinostat, vorozole, yttrium-90 glass microspheres, zinostatin, zinostatin stimalamer, zoledronic acid, zorubicin, or a combination thereof.

The additional pharmaceutical agent can be afinitor, aldesleukin, alendronic acid, alfaferone, alitretinoin, allopurinol, aloprim, aloxi, altretamine, aminoglutethimide, amifostine, amrubicin, amsacrine, anastrozole, anzmet, aranesp, arglabin, arsenic trioxide, aromasin, 5-azacytidine, azathioprine, copanlisib (BAY 80-6946), BCG or tice BCG, bestatin, betamethasone acetate, betamethasone sodium phosphate, bexarotene, bleomycin sulfate, broxuridine, bortezomib, busulfan, calcitonin, campath, capecitabine, carboplatin, casodex, cefesone, celmoleukin, cerubidine, chlorambucil, cisplatin, cladribine, clodronic acid, cyclophosphamide, cytarabine, dacarbazine, dactinomycin, DaunoXome, decadron, decadron phosphate, delestrogen, denileukin diftitox, depomedrol, deslorelin, dexrazoxane, diethylstilbestrol, diflucan, docetaxel, doxifluridine, doxorubicin, dronabinol, DW-166HC, eligard, elitek, ellence, emend, epirubicin, epoetin alfa, epogen, eptaplatin, ergamisol, estrace, estradiol, estramustine phosphate sodium, ethinyl estradiol, ethyol, etidronic acid, etopophos, etoposide, fadrozole, farston, filgrastim, finasteride, fligrastim, floxuridine, fluconazole, fludarabine, 5-fluorodeoxyuridine monophosphate, 5-fluorouracil (5-FU), fluoxymesterone, flutamide, formestane, fosteabine, fotemustine, fulvestrant, gammagard, gemcitabine, gemtuzumab, gleevec, gliadel, goserelin, granisetron HCl, histrelin, hycamtin, hydrocortone, eyrthro-hydroxynonyladenine, hydroxyurea, ibritumomab tiuxetan, idarubicin, ifosfamide, interferon alpha, interferon-alpha 2, interferon alfa-2A, interferon alfa-2B, interferon alfa-n1, interferon alfa-n3, interferon beta, interferon gamma-1a, interleukin-2, intron A, iressa, irinotecan, kytril, lapatinib, lentinan sulfate, letrozole, leucovorin, leuprolide, leuprolide acetate, levamisole, levofolinic acid calcium salt, levothroid, levoxyl, lomustine, lonidamine, marinol, mechlorethamine, mecobalamin, medroxyprogesterone acetate, megestrol acetate, melphalan, menest, 6-mercaptopurine, Mesna, methotrexate, metvix, miltefosine, minocycline, mitomycin C, mitotane, mitoxantrone, Modrenal, Myocet, nedaplatin, neulasta, neumega, neupogen, nilutamide, nolvadex, NSC-631570, OCT-43, octreotide, ondansetron HCl, orapred, oxaliplatin, paclitaxel, pediapred, pegaspargase, Pegasys, pentostatin, picibanil, pilocarpine HCl, pirarubicin, plicamycin, porfimer sodium, prednimustine, prednisolone, prednisone, premarin, procarbazine, procrit, raltitrexed, rebif, rhenium-186 etidronate, rituximab, roferon-A, romurtide, salagen, sandostatin, sargramostim, semustine, sizofiran, sobuzoxane, solu-medrol, sparfosic acid, stem-cell therapy, streptozocin, strontium-89 chloride, sunitinib, synthroid, tamoxifen, tamsulosin, tasonermin, tastolactone, taxotere, teceleukin, temozolomide, teniposide, testosterone propionate, testred, thioguanine, thiotepa, thyrotropin, tiludronic acid, topotecan, toremifene, tositumomab, trastuzumab, treosulfan, tretinoin, trexall, trimethylmelamine, trimetrexate, triptorelin acetate, triptorelin pamoate, UFT, uridine, valrubicin, vesnarinone, vinblastine, vincristine, vindesine, vinorelbine, virulizin, zinecard, zinostatin stimalamer, zofran, ABI-007, acolbifene, actimmune, affinitak, aminopterin, arzoxifene, asoprisnil, atamestane, atrasentan, sorafenib (BAY 43-9006), avastin, CCI-779, CDC-501, celebrex, cetuximab, crisnatol, cyproterone acetate, decitabine, DN-101, doxorubicin-MTC, dSLIM, dutasteride, edotecarin, eflornithine, exatecan, fenretinide, histamine dihydrochloride, histrelin hydrogel implant, holmium-166 DOTMP, ibandronic acid, interferon gamma, intron-PEG, ixabepilone, keyhole limpet hemocyanin, L-651582, lanreotide, lasofoxifene, libra, lonafarnib, miproxifene, minodronate, MS-209, liposomal MTP-PE, MX-6, nafarelin, nemorubicin, neovastat, nolatrexed, oblimersen, onco-TCS, osidem, paclitaxel polyglutamate, pamidronate disodium, PN-401, QS-21, quazepam, R-1549, raloxifene, ranpirnase, 13-cis -retinoic acid, satraplatin, seocalcitol, T-138067, tarceva, taxoprexin, thymosin alpha 1, tiazofurine, tipifarnib, tirapazamine, TLK-286, toremifene, TransMID-107R, valspodar, vapreotide, vatalanib, verteporfin, vinflunine, Z-100, zoledronic acid or combinations thereof.

Optional anti-hyper-proliferative agents which can be added to the composition include but are not limited to compounds listed on the cancer chemotherapy drug regimens in the 11th Edition of the Merck Index, (1996), which is hereby incorporated by reference, such as asparaginase, bleomycin, carboplatin, carmustine, chlorambucil, cisplatin, colaspase, cyclophosphamide, cytarabine, dacarbazine, dactinomycin, daunorubicin, doxorubicin (adriamycine), epirubicin, epothilone, an epothilone derivative, etoposide, 5-fluorouracil, hexamethylmelamine, hydroxyurea, ifosfamide, irinotecan, leucovorin, lomustine, mechlorethamine, 6-mercaptopurine, mesna, methotrexate, mitomycin C, mitoxantrone, prednisolone, prednisone, procarbazine, raloxifene, streptozocin, tamoxifen, thioguanine, topotecan, vinblastine, vincristine, and vindesine.

Other anti-hyper-proliferative agents suitable for use with the composition of the invention include but are not limited to those compounds acknowledged to be used in the treatment of neoplastic diseases in Goodman and Gilman's The Pharmacological Basis of Therapeutics (Ninth Edition), editor Molinoff et al., publ. by McGraw-Hill, pages 1225-1287, (1996), which is hereby incorporated by reference, such as aminoglutethimide, L-asparaginase, azathioprine, 5-azacytidine cladribine, busulfan, diethylstilbestrol, 2',2'-difluorodeoxycytidine, docetaxel, erythrohydroxynonyl adenine, ethinyl estradiol, 5-fluorodeoxyuridine, 5-fluorodeoxyuridine monophosphate, fludarabine phosphate, fluoxymesterone, flutamide, hydroxyprogesterone caproate, idarubicin, interferon, medroxyprogesterone acetate, megestrol acetate, melphalan, mitotane, paclitaxel, pentostatin, N-phosphonoacetyl-L-aspartate (PALA), plicamycin, semustine, teniposide, testosterone propionate, thiotepa, trimethylmelamine, uridine, and vinorelbine.

Other anti-hyper-proliferative agents suitable for use with the composition of the invention include but are not limited to other anti-cancer agents such as epothilone and its derivatives, irinotecan, raloxifene and topotecan.

The coated tablets of the present invention may also be administered in combination with protein therapeutics. Such protein therapeutics suitable for the treatment of cancer or other angiogenic disorders and for use with the compositions of the invention include, but are not limited to, an interferon (e.g., interferon .alpha., .beta., or .gamma.) supraagonistic monoclonal antibodies, Tuebingen, TRP-1 protein vaccine, Colostrinin, anti-FAP antibody, YH-16, gemtuzumab, infliximab, cetuximab, trastuzumab, denileukin diftitox, rituximab, thymosin alpha 1, bevacizumab, mecasermin, mecasermin rinfabate, oprelvekin, natalizumab, rhMBL, MFE-CP1 + ZD-2767-P, ABT-828, ErbB2-specific immunotoxin, SGN-35, MT-103, rinfabate, AS-1402, B43-genistein, L-19 based radioimmunotherapeutics, AC-9301, NY-ESO-1 vaccine, IMC-1C11, CT-322, rhCC10, r(m)CRP, MORAb-009, aviscumine, MDX-1307, Her-2 vaccine, APC-8024, NGR-hTNF, rhH1.3, IGN-311, Endostatin, volociximab, PRO-1762, lexatumumab, SGN-40, pertuzumab, EMD-273063, L19-IL-2 fusion protein, PRX-321, CNTO-328, MDX-214, tigapotide, CAT-3888, labetuzumab, alpha-particle-emitting radioisotope-Ilinked lintuzumab, EM-1421, HyperAcute vaccine, tucotuzumab celmoleukin, galiximab, HPV-16-E7, Javelin - prostate cancer, Javelin - melanoma, NY-ESO-1 vaccine, EGF vaccine, CYT-004-MelQbG10, WT1 peptide, oregovomab, ofatumumab, zalutumumab, cintredekin besudotox, WX-G250, Albuferon, aflibercept, denosumab, vaccine, CTP-37, efungumab, or 131I-chTNT-1/B. Monoclonal antibodies useful as the protein therapeutic include, but are not limited to, muromonab-CD3, abciximab, edrecolomab, daclizumab, gentuzumab, alemtuzumab, ibritumomab, cetuximab, bevicizumab, efalizumab, adalimumab, omalizumab, muromomab-CD3, rituximab, daclizumab, trastuzumab, palivizumab, basiliximab, and infliximab.

The coated tablets of the present invention may also be combined with biological therapeutic agents, such as antibodies (e.g. avastin, rituxan, erbitux, herceptin), or recombinant proteins.

In accordance with an embodiment, the present invention relates to pharmaceutical combinations comprising :
- a coated tablet of the present invention as defined herein ;
   and
- one or more agents selected from : a taxane, such as Docetaxel, Paclitaxel, lapatinib, sunitinib, or Taxol; an epothilone, such as Ixabepilone, Patupilone, or Sagopilone; Mitoxantrone; Predinisolone; Dexamethasone; Estramustin; Vinblastin; Vincristin; Doxorubicin; Adriamycin; Idarubicin; Daunorubicin; Bleomycin; Etoposide; Cyclophosphamide; Ifosfamide; Procarbazine; Melphalan; 5-Fluorouracil; Capecitabine; Fludarabine; Cytarabine; Ara-C; 2-Chloro-2'-deoxyadenosine; Thioguanine; an anti-androgen, such as Flutamide, Cyproterone acetate, or Bicalutamide; Bortezomib; a platinum derivative, such as Cisplatin, or Carboplatin; Chlorambucil; Methotrexate; and Rituximab.

The coated tablets of the present invention may also be in combination with antiangiogenesis agents, such as, for example, with avastin, axitinib, DAST, recentin, sorafenib or sunitinib. Combinations with inhibitors of proteasomes or mTOR inhibitors, or anti-hormones or steroidal metabolic enzyme inhibitors are also possible.

Generally, the use of cytotoxic and/or cytostatic agents in combination with a coated tablet of the present invention will serve to:
(1) yield better efficacy in reducing the growth of a tumour or even eliminate the tumour as compared to administration of either agent alone,
(2) provide for the administration of lesser amounts of the administered chemotherapeutic agents,
(3) provide for a chemotherapeutic treatment that is well tolerated in the patient with fewer deleterious pharmacological complications than observed with single agent chemotherapies and certain other combined therapies,
(4) provide for treating a broader spectrum of different cancer types in mammals, especially humans,
(5) provide for a higher response rate among treated patients,
(6) provide for a longer survival time among treated patients compared to standard chemotherapy treatments,
(7) provide a longer time for tumour progression, and/or
(8) yield efficacy and tolerability results at least as good as those of the agents used alone, compared to known instances where other cancer agent combinations produce antagonistic effects.

### Methods of Sensitizing Cells to Radiation

In a distinct embodiment of the present invention, a coated tablet of the present invention may be used to sensitize a cell to radiation. That is, treatment of a cell with a compound of the present invention prior to radiation treatment of the cell renders the cell more susceptible to DNA damage and cell death than the cell would be in the absence of any treatment with a compound of the invention. In one aspect, the cell is treated with at least one compound of the invention.

Thus, the present invention also provides a method of killing a cell, wherein a cell is administered with a coated one or more compounds of the invention in combination with conventional radiation therapy.

The present invention also provides a method of rendering a cell more susceptible to cell death, wherein the cell is treated with one or more compounds of the invention prior to the treatment of the cell to cause or induce cell death. In one aspect, after the cell is treated with one or more compounds of the invention, the cell is treated with at least one compound, or at least one method, or a combination thereof, in order to cause DNA damage for the purpose of inhibiting the function of the normal cell or killing the cell.

In one embodiment, a cell is killed by treating the cell with at least one DNA damaging agent. That is, after treating a cell with one or more compounds of the invention to sensitize the cell to cell death, the cell is treated with at least one DNA damaging agent to kill the cell. DNA damaging agents useful in the present invention include, but are not limited to, chemotherapeutic agents (e.g., cisplatinum), ionizing radiation (X-rays, ultraviolet radiation), carcinogenic agents, and mutagenic agents.

In another embodiment, a cell is killed by treating the cell with at least one method to cause or induce DNA damage. Such methods include, but are not limited to, activation of a cell signalling pathway that results in DNA damage when the pathway is activated, inhibiting of a cell signalling pathway that results in DNA damage when the pathway is inhibited, and inducing a biochemical change in a cell, wherein the change results in DNA damage. By way of a non-limiting example, a DNA repair pathway in a cell can be inhibited, thereby preventing the repair of DNA damage and resulting in an abnormal accumulation of DNA damage in a cell.

In one aspect of the invention, a compound of the invention is administered to a cell prior to the radiation or other induction of DNA damage in the cell. In another aspect of the invention, a compound of the invention is administered to a cell concomitantly with the radiation or other induction of DNA damage in the cell. In yet another aspect of the invention, a compound of the invention is administered to a cell immediately after radiation or other induction of DNA damage in the cell has begun.

In another aspect, the cell is in vitro. In another embodiment, the cell is in vivo.

As mentioned supra, the compounds of the present invention have surprisingly been found to effectively inhibit MKNK-1 and may therefore be used for the treatment or prophylaxis of diseases of uncontrolled cell growth, proliferation and/or survival, inappropriate cellular immune responses, or inappropriate cellular inflammatory responses, or diseases which are accompanied with uncontrolled cell growth, proliferation and/or survival, inappropriate cellular immune responses, or inappropriate cellular inflammatory responses, particularly in which the uncontrolled cell growth, proliferation and/or survival, inappropriate cellular immune responses, or inappropriate cellular inflammatory responses is mediated by MKNK-1, such as, for example, haematological tumours, solid tumours, and/or metastases thereof, e.g. leukaemias and myelodysplastic syndrome, malignant lymphomas, head and neck tumours including brain tumours and brain metastases, tumours of the thorax including non-small cell and small cell lung tumours, gastrointestinal tumours, endocrine tumours, mammary and other gynaecological tumours, urological tumours including renal, bladder and prostate tumours, skin tumours, and sarcomas, and/or metastases thereof.

In accordance with another aspect therefore, the present invention covers a coated tablet of the present invention as defined herein for use in the treatment or prophylaxis of a disease, as mentioned supra.

Another particular aspect of the present invention is therefore the use of a coated tablet of the present invention as defined herein, for the prophylaxis or treatment of a disease.

The diseases referred to in the two preceding paragraphs are diseases of uncontrolled cell growth, proliferation and/or survival, inappropriate cellular immune responses, or inappropriate cellular inflammatory responses, or diseases which are accompanied with uncontrolled cell growth, proliferation and/or survival, inappropriate cellular immune responses, or inappropriate cellular inflammatory responses, particularly in which the uncontrolled cell growth, proliferation and/or survival, inappropriate cellular immune responses, or inappropriate cellular inflammatory responses is mediated by allo-MEK, such as, for example, haematological tumours, solid tumours, and/or metastases thereof, e.g. leukaemias and myelodysplastic syndrome, malignant lymphomas, head and neck tumours including brain tumours and brain metastases, tumours of the thorax including non-small cell and small cell lung tumours, gastrointestinal tumours, endocrine tumours, mammary and other gynaecological tumours, urological tumours including renal, bladder and prostate tumours, skin tumours, and sarcomas, and/or metastases thereof.

The term "inappropriate" within the context of the present invention, in particular in the context of "inappropriate cellular immune responses, or inappropriate cellular inflammatory responses", as used herein, is to be understood as preferably meaning a response which is less than, or greater than normal, and which is associated with, responsible for, or results in, the pathology of said diseases.

Preferably, the use is in the treatment or prophylaxis of diseases, wherein the diseases are haemotological tumours, solid tumours and/or metastases thereof.

### Method of treating hyper-proliferative disorders

The present invention relates to a method for using the coated tablets of the present invention as defined herein, to treat mammalian hyper-proliferative disorders. Said coated tablets can be utilized to inhibit, block, reduce, decrease, etc., cell proliferation and/or cell division, and/or produce apoptosis. This method comprises administering to a mammal in need thereof, including a human, a coated tablet of this invention, or a pharmaceutically acceptable salt, isomer, polymorph, metabolite, hydrate, solvate or ester thereof; etc. which is effective to treat the disorder. Hyper-proliferative disorders include but are not limited, e.g., psoriasis, keloids, and other hyperplasias affecting the skin, benign prostate hyperplasia (BPH), solid tumours, such as cancers of the breast, respiratory tract, brain, reproductive organs, digestive tract, urinary tract, eye, liver, skin, head and neck, thyroid, parathyroid and their distant metastases. Those disorders also include lymphomas, sarcomas, and leukaemias.

Examples of breast cancer include, but are not limited to invasive ductal carcinoma, invasive lobular carcinoma, ductal carcinoma in situ, and lobular carcinoma in situ.

Examples of cancers of the respiratory tract include, but are not limited to small-cell and non-small-cell lung carcinoma, as well as bronchial adenoma and pleuropulmonary blastoma.

Examples of brain cancers include, but are not limited to brain stem and hypophtalmic glioma, cerebellar and cerebral astrocytoma, medulloblastoma, ependymoma, as well as neuroectodermal and pineal tumour.

Tumours of the male reproductive organs include, but are not limited to prostate and testicular cancer. Tumours of the female reproductive organs include, but are not limited to endometrial, cervical, ovarian, vaginal, and vulvar cancer, as well as sarcoma of the uterus.

Tumours of the digestive tract include, but are not limited to anal, colon, colorectal, oesophageal, gallbladder, gastric, pancreatic, rectal, small-intestine, and salivary gland cancers.

Tumours of the urinary tract include, but are not limited to bladder, penile, kidney, renal pelvis, ureter, urethral and human papillary renal cancers.

Eye cancers include, but are not limited to intraocular melanoma and retinoblastoma.

Examples of liver cancers include, but are not limited to hepatocellular carcinoma (liver cell carcinomas with or without fibrolamellar variant), cholangiocarcinoma (intrahepatic bile duct carcinoma), and mixed hepatocellular cholangiocarcinoma.

Skin cancers include, but are not limited to squamous cell carcinoma, Kaposi's sarcoma, malignant melanoma, Merkel cell skin cancer, and non-melanoma skin cancer.

Head-and-neck cancers include, but are not limited to laryngeal, hypopharyngeal, nasopharyngeal, oropharyngeal cancer, lip and oral cavity cancer and squamous cell. Lymphomas include, but are not limited to AIDS-related lymphoma, non-Hodgkin's lymphoma, cutaneous T-cell lymphoma, Burkitt lymphoma, Hodgkin's disease, and lymphoma of the central nervous system.

Sarcomas include, but are not limited to sarcoma of the soft tissue, osteosarcoma, malignant fibrous histiocytoma, lymphosarcoma, and rhabdomyosarcoma.

Leukemias include, but are not limited to acute myeloid leukemia, acute lymphoblastic leukemia, chronic lymphocytic leukemia, chronic myelogenous leukemia, and hairy cell leukemia.

These disorders have been well characterized in humans, but also exist with a similar etiology in other mammals, and can be treated by administering pharmaceutical compositions of the present invention.

The term "treating" or "treatment" as stated throughout this document is used conventionally, *e.g*., the management or care of a subject for the purpose of combating, alleviating, reducing, relieving, improving the condition of, *etc.,* of a disease or disorder, such as a carcinoma.

### Methods of treating kinase disorders

The present invention also provides methods for the treatment of disorders associated with aberrant mitogen extracellular kinase activity, including, but not limited to stroke, heart failure, hepatomegaly, cardiomegaly, diabetes, Alzheimer's disease, cystic fibrosis, symptoms of xenograft rejections, septic shock or asthma.

Effective amounts of coated tablets of the present invention can be used to treat such disorders, including those diseases (*e.g*., cancer) mentioned in the background section above. Nonetheless, such cancers and other diseases can be treated with coated tablets of the present invention, regardless of the mechanism of action and/or the relationship between the kinase and the disorder.

The phrase "aberrant kinase activity" or "aberrant tyrosine kinase activity," includes any abnormal expression or activity of the gene encoding the kinase or of the polypeptide it encodes. Examples of such aberrant activity, include, but are not limited to, over-expression of the gene or polypeptide ; gene amplification; mutations which produce constitutively-active or hyperactive kinase activity; gene mutations, deletions, substitutions, additions, etc.

The present invention also provides for methods of inhibiting a kinase activity, especially of mitogen extracellular kinase, comprising administering an effective amount of coated tablets of the present invention, including salts, polymorphs, metabolites, hydrates, solvates, prodrugs (*e.g*.: esters) thereof, and diastereoisomeric forms thereof. Kinase activity can be inhibited in cells (*e.g., in vitro*), or in the cells of a mammalian subject, especially a human patient in need of treatment.

### Methods of treating angiogenic disorders

The present invention also provides methods of treating disorders and diseases associated with excessive and/or abnormal angiogenesis.

Inappropriate and ectopic expression of angiogenesis can be deleterious to an organism. A number of pathological conditions are associated with the growth of extraneous blood vessels. These include, e.g., diabetic retinopathy, ischemic retinal-vein occlusion, and retinopathy of prematurity [Aiello et al. New Engl. J. Med. 1994, 331, 1480; Peer et al. Lab. Invest. 1995, 72, 638], age-related macular degeneration [AMD; see, Lopez et al. Invest. Opththalmol. Vis. Sci. 1996, 37, 855], neovascular glaucoma, psoriasis, retrolental fibroplasias, angiofibroma, inflammation, rheumatoid arthritis (RA), restenosis, in-stent restenosis, vascular graft restenosis, etc. In addition, the increased blood supply associated with cancerous and neoplastic tissue, encourages growth, leading to rapid tumour enlargement and metastasis. Moreover, the growth of new blood and lymph vessels in a tumour provides an escape route for renegade cells, encouraging metastasis and the consequence spread of the cancer. Thus, coated tablets of the present invention can be utilized to treat and/or prevent any of the aforementioned angiogenesis disorders, e.g., by inhibiting and/or reducing blood vessel formation; by inhibiting, blocking, reducing, decreasing, etc. endothelial cell proliferation or other types involved in angiogenesis, as well as causing cell death or apoptosis of such cell types.

### Dose and administration

Based upon standard laboratory techniques known to evaluate compounds useful for the treatment of hyper-proliferative disorders and angiogenic disorders, by standard toxicity tests and by standard pharmacological assays for the determination of treatment of the conditions identified above in mammals, and by comparison of these results with the results of known medicaments that are used to treat these conditions, the effective dosage of the coated tablets of this invention can readily be determined for treatment of each desired indication. The amount of the active ingredient to be administered in the treatment of one of these conditions can vary widely according to such considerations as the particular coated tablets and dosage unit employed, the mode of administration, the period of treatment, the age and sex of the patient treated, and the nature and extent of the condition treated.

The total amount of refametinib to be administered orally will generally range from about 0.001 mg/kg to about 200 mg/kg body weight per day, and preferably from about 0.01 mg/kg to about 20 mg/kg body weight per day. Clinically useful dosing schedules will range from one to three times a day dosing to once every four weeks dosing. In addition, "drug holidays" in which a patient is not dosed with a drug for a certain period of time, may be beneficial to the overall balance between pharmacological effect and tolerability. A unit dosage may contain from about 0.5 mg to about 1500 mg of active ingredient, and can be administered one or more times per day or less than once a day. The average daily dosage for administration by injection, including intravenous, intramuscular, subcutaneous and parenteral injections, and use of infusion techniques will preferably be from 0.01 to 200 mg/kg of total body weight. The average daily rectal dosage regimen will preferably be from 0.01 to 200 mg/kg of total body weight. The average daily vaginal dosage regimen will preferably be from 0.01 to 200 mg/kg of total body weight. The average daily topical dosage regimen will preferably be from 0.1 to 200 mg administered between one to four times daily. The transdermal concentration will preferably be that required to maintain a daily dose of from 0.01 to 200 mg/kg. The average daily inhalation dosage regimen will preferably be from 0.01 to 100 mg/kg of total body weight.

Of course the specific initial and continuing dosage regimen for each patient will vary according to the nature and severity of the condition as determined by the attending diagnostician, the activity of the specific compound employed, the age and general condition of the patient, time of administration, route of administration, rate of excretion of the drug, drug combinations, and the like. The desired mode of treatment and number of doses of a coated tablet of the present invention can be ascertained by those skilled in the art using conventional treatment tests.

Preferably, the diseases of said method are haematological tumours, solid tumour and/or metastases thereof.

The coated tablets of the present invention can be used in particular in therapy and prevention, i.e. prophylaxis, of tumour growth and metastases, especially in solid tumours of all indications and stages with or without pre-treatment of the tumour growth.

## Claims

1. A coated tablet comprising :
• a core, comprising :
∘ refametinib, a hydrate, solvate, polymorph, or a pharmaceutically acceptable salt thereof; and
∘ one or more pharmaceutically acceptable core excipients; and
• a polyethylene glycol-free, stability-maintaining coating, comprising:
∘ a stability-maintaining film former, which is hydroxypropyl methylcellulose ;
∘ optionally, a further stability-maintaining film former;
∘ and, optionally,
∘ one or more pharmaceutically acceptable coating excipients.

2. The coated tablet according to claim 1, **characterised in that** said further stability-maintaining film former is selected from the group consisting of : hydroxyethyl cellulose, hydroxypropyl cellulose, methyl cellulose, polyvinyl alcohol (PVA), polyvinylpyrrolidone, a copolymer of vinylpyrrolidone and vinylacetate, a copolymer of vinylpyrrolidone and vinylacetate sucrose, polyacrylate and polymethacrylate, a copolymer of dimethylaminomethacrylic acid and a neutral methacrylic acid ester, a polymer of methacrylic acid or methacrylic acid esters, a copolymer of acrylic acid ethyl ester and methacrylic acid methyl ester, a copolymer of methacrylic acid and acrylic acid methyl ester, a copolymer of acrylic acid and acrylic acid methyl ester, liquid glucose, ethyl cellulose, cellulose acetate phthalate, hydroxypropyl methyl cellulose acetate succinate, hydroxypropyl methyl cellulose phthalate, shellac, and mixtures thereof, preferably polyvinyl alcohol (PVA).

3. The coated tablet according to any one of claims 1 or 2, **characterised in that** said hydroxypropyl methylcellulose is present in an amount of 30 to 100%, preferably 40 to 55%, by weight of said polyethylene glycol-free, stability-maintaining coating.

4. A coated tablet comprising:
• a core, comprising:
∘ refametinib, a hydrate, solvate, polymorph, or a pharmaceutically acceptable salt thereof; and
∘ one or more pharmaceutically acceptable core excipients; and
• a stability-maintaining coating, comprising:
∘ a stability-maintaining film former, which is polyvinyl alcohol, particularly partially hydrolysed polyvinyl alcohol;
∘ optionally, a further stability-maintaining film former;
∘ a plasticizer, which is polyethylene glycol;
∘ and, optionally,
∘ one or more pharmaceutically acceptable coating excipients.

5. The coated tablet according to claim 1 or 4, **characterised in that** said refametinib is in micronized form.

6. The coated tablet according to claim 1 or 4, **characterised in that** said refametinib is in the form of polymorph A.

7. The coated tablet according to claim 4, **characterised in that** said stability-maintaining film former is polyvinyl alcohol (partially hydrolysed) (PVA) and is present in an amount of 40 to 55%, preferably by weight of said stability-maintaining coating.

8. The coated tablet according to any one of claims 4 or 7, **characterised in that** said further stability-maintaining film former is selected from the group consisting of: hydroxyethyl cellulose, hydroxypropyl cellulose, methyl cellulose, polyvinylpyrrolidone, a copolymer of vinylpyrrolidone and vinylacetate, a copolymer of vinylpyrrolidone and vinylacetate sucrose, polyacrylate and polymethacrylate, a copolymer of dimethylaminomethacrylic acid and a neutral methacrylic acid ester, a polymer of methacrylic acid or methacrylic acid esters, a copolymer of acrylic acid ethyl ester and methacrylic acid methyl ester, a copolymer of methacrylic acid and acrylic acid methyl ester, a copolymer of acrylic acid and acrylic acid methyl ester, liquid glucose, ethyl cellulose, cellulose acetate phthalate, hydroxypropyl methyl cellulose acetate succinate, hydroxypropyl methyl cellulose phthalate, shellac, and mixtures thereof.

9. The coated tablet according to any one of claims 1 to 8, **characterised in that** said one or more pharmaceutically acceptable coating excipients present in said stability-maintaining coating is (are) selected from the group consisting of:
* one or more plasticizers, selected from the group consisting of: propylene glycol, polypropylene glycol, sorbitol, glycerol, maltitol, xylitol, mannitol, erythritol, glycerol trioleate, tributyl citrate, triethyl citrate, acetyl triethyl citrate, glyceryl triacetate, stearic acid, medium chain triglycerides, and mixtures thereof;
* one or more colorants selected from the group consisting of: ferric oxide red, ferric oxide yellow, ferric oxide black, titanium dioxide, indigotine, sunset yellow FCF, tartrazin, erythrosine, quinoline yellow, carbon black, anthocyanin, riboflavin, carmine, curcumin, chlorophyll, carotene, and mixtures thereof, preferably titanium dioxide and a ferric oxide, such as ferric oxide yellow, ferric oxide red and/or ferric oxide black, more preferably a ferric oxide and titanium dioxide;
* one or more anti-tacking agents selected from the group consisting of: talc, magnesium stearate, stearic acid, lecithin, soy lecithin, mineral oil, carnauba wax, acetylated monoglycerides and/or polysorbate and mixtures thereof, preferably talc, lecithin, soy lecithin and/or polysorbate, and mixtures thereof, more preferably talc, lecithin and soy lecithin; and
* one or more opacifiers selected from the group consisting of: talc and titanium dioxide.

10. The coated tablet according to any one of claims 1 to 9, **characterised in that** said one or more pharmaceutically acceptable core excipient(s) present in said core is (are) selected from the group consisting of:
* one or more fillers or dry binders selected from the group consisting of: powdered cellulose, microcrystalline cellulose, silicified microcrystalline cellulose, ethylcellulose, dicalcium phosphate, tricalcium phosphate, kaolin, magnesium trisilicate, mannitol, sorbitol, maltitol, xylitol, lactose, dextrose, maltose, sucrose, glucose, fructose, maltodextrines, precipitated calcium carbonate, sodium carbonate, sodium phosphate and starch, preferably microcrystalline cellulose, mannitol, and/or lactose, more preferably mannitol, microcrystalline cellulose and/or lactose;
* one or more disintegrants selected from the group consisting of: croscarmellose sodium, crospovidone (cross-linked polyvinylpyrrolidone), sodium starch glycollate, carboxymethylcellulose calcium, carboxymethylcellulose sodium, microcrystalline cellulose, hydroxypropyl cellulose, low substituted hydroxypropyl cellulose, polacrillin potassium, alginic acid, sodium alginate, partially hydrolysed starch, sodium carboxymethyl starch and starch, preferably croscarmellose sodium and/or crospovidone, more preferably croscarmellose sodium;
* one or more surfactants, particularly a wetting agent selected from the group consisting of: heptadecaethylene oxycetanol, lecithins, polyoxyethylene stearate, nonoxynol 9, nonoxynol 10, oxtoxynol 9, sorbitan fatty acid esters, polysorbates, sodium salts of fatty alcohol sulfates, sodium salts of sulfosuccinates, partially esters of fatty acids with alcohols such as glycerine monostearate, ethers of fatty alcohols with polyoxyethylene, esters of fatty acids with polyoxyethylene, copolymers of ethylenoxide and propylenoxide (Pluronic^{®}), benzalkonium chloride and ethoxylated triglycerides, preferably sodium lauryl sulfate;
* one or more binders (for wet granulation) selected from the group consisting of: hydroxypropyl cellulose, hypromellose (hydroxypropyl methylcellulose, HPMC), hydroxyethylcellulose, carboxymethylcellulose, carboxymethylcellulose calcium, carboxymethylcellulose sodium, ethylcellulose, methylcellulose, povidone (polyvinylpyrrolidone, PVP), polyvinyl alcohol, polyacrylates, gelatine, liquid glucose, arabic gum, alginic acid and pregelatinized starch, preferably a hydrophilic binder which is soluble in the aqueous granulation liquid, more preferably hypromellose (hydroxypropyl methylcellulose, HPMC) and/or polyvinylpyrrolidone, more preferably still hypromellose;
* one or more lubricants selected from the group consisting of: calcium stearate, magnesium stearate, zinc stearate, stearic acid, fumaric acid, sodium stearylfumarate, glycerol dibehenate, glycerol distearate, glyceryl dipalmitostearate, behenoyl polyoxyl-8 glycerides, mineral oil, and polyethylene glycol, preferably magnesium stearate.

11. The coated tablet according to any one of claims 1, 2, 3, 9, or 10, which comprises:
• a core, comprising:
∘ refametinib, which is micronized polymorph A; and
∘ pharmaceutically acceptable core excipients, which are:
• a filler and dry binder, which is mannitol, which preferably is present in an amount of 0 to 95%, more preferably 30 to 80%, by weight of the total weight of said core;
• a disintegrant, which is crocarmellose sodium, preferably contained in an amount of 0 to 15%, more preferably 3 to 10%, by weight of the total weight of said core ;
• a surfactant, which is sodium lauryl sulfate, which preferably is present in an amount of 0.5 to 1%, more preferably 0.1 to 2%, by weight of the total weight of said core;
• a binder, which is hypromellose, which preferably is present in an amount of 0 to 15%, more preferably 0.5 to 8%, by weight of the total weight of said core;
• a lubricant, which is magnesium stearate, preferably contained in an amount of 0 to 5%, more preferably 0.2 to 3%, by weight of the total weight of said core;
and
• a polyethylene glycol-free, stability-maintaining coating, comprising:
∘ a stability-maintaining film former, which is:
▪ hydroxypropyl methylcellulose (hypromellose (HPMC)), preferably present in an amount of 30 to 100%, more preferably 40 to 55%, by weight of said polyethylene glycol-free, stability-maintaining coating; and, optionally,
∘ pharmaceutically acceptable coating excipients, which are:
• a colorant, which is a ferric oxide or titanium dioxide, preferably present in an amount of 10 to 50% by weight of said polyethylene glycol-free, stability-maintaining coating;
• an anti-tacking agent, which is talc, preferably present in an amount of 1 to 40%, more preferably 3 to 25%, by weight of said polyethylene glycol-free, stability-maintaining coating; and
• an opacifier, which is talc and/or titanium dioxide, preferably present in an amount of 10 to 60% by weight of said polyethylene glycol-free, stability-maintaining coating.

12. The coated tablet according to any one of claims 4, 7, 9, or 10, which comprises:
• a core, comprising:
∘ refametinib, which is micronized polymorph A; and
∘ pharmaceutically acceptable core excipients, which are:
• a filler and dry binder, which is mannitol, which preferably is present in an amount of 0 to 95%, more preferably 30 to 80%, by weight of the total weight of said core;
• a disintegrant, which is crocarmellose sodium, preferably contained in an amount of 0 to 15%, more preferably 3 to 10%, by weight of the total weight of said core;
• a surfactant, which is sodium lauryl sulfate, which preferably is present in an amount of 0.5 to 1%, more preferably 0.1 to 2%, by weight of the total weight of said core;
• a binder, which is hypromellose, which preferably is present in an amount of 0 to 15%, more preferably 0.5 to 8%, by weight of the total weight of said core;
• a lubricant, which is magnesium stearate, preferably contained in an amount of 0 to 5%, more preferably 0.2 to 3%, by weight of the total weight of said core;
and
• a stability-maintaining coating, comprising:
∘ a stability-maintaining film former, which is:
▪ polyvinyl alcohol (partially hydrolysed) (PVA), preferably present in an amount of 30 to 100%, more preferably 40 to 55%, by weight of said stability-maintaining coating;
▪ polyethylene glycol (PEG), preferably present in an amount of 0 to 19% by weight of said stability-maintaining coating;
and, optionally,
∘ pharmaceutically acceptable coating excipients, which are:
• a colorant, which is a ferric oxide or titanium dioxide, preferably present in an amount of 10 to 50% by weight of said stability-maintaining coating;
• an anti-tacking agent, which is talc, preferably present in an amount of 1 to 40%, more preferably 3 to 25%, by weight of said stability-maintaining coating; and
• an opacifier, which is talc and/or titanium dioxide, preferably present in an amount of 10 to 60% by weight of said stability-maintaining coating.

13. The coated tablet according to any one of claims 1 to 12, which contains 10, 20, 30 or 50 mg refametinib.

14. A method of preparing a coated tablet according to any one of claims 1 to 13, comprising the following steps:
a) granulation, in which:
i) said binder and said wetting agent are placed in water and dissolved, thus providing a granulation liquid;
ii) said granulation liquid is applied, such as by spraying for example, onto a mixture of micronized polymorph A refametinib, said filler/dry binder and said disintegrant, as a powder bed for example, in a fluidized bed granulator for example, at a temperature such as of 20 to 35°C for example, preferably of 28 to 34°C;
thus providing a refametinib-containing granulate;
b) tableting, in which:
i) said granulate is screened to a suitable size, such as 0.5 to 1.0 mm for example, preferably 0.8 mm, thus providing a screened refametinib-containing granulate;
ii) blending said screened refametinib-containing granulate with said disintegrant and said lubricant, thus providing a ready-to-press refametinib-containing blend;
iii) compressing said refametinib-containing blend into tablets, such as on a rotary tablet press for example, thus providing refametinib-containing uncoated tablets, containing 10, 20, 30 or 50 mg refametinib, for example;
c) film coating, in which:
i) dispersing said stability-maintaining film former, said anti-tacking agent and said colorants as well as optionally said plasticizers in water, thus providing a coating dispersion;
ii) applying, such as by spraying for example, said coating dispersion onto said refametinib-containing uncoated tablets, such as in a perforated drum coater for example, at a temperature of 35 to 60°C for example, preferably of 40 to 55°C;
thus providing coated tablets according to any one of claims 1 to 13.

15. The coated tablet according to any one of claims 1 to 13 for use in the treatment or prophylaxis of a disease, in particular cancer, such as hepatocellular carcinoma, pancreatic cancer, colorectal cancer, non-small cell lung carcinoma, non-Hodgkin's lymphoma or breast cancer; optionally in combination with one or more active agents, such as, anti-hyper-proliferative or cytotoxic agents, signal transduction inhibitors, or with other anti-cancer agents or therapies, or other indication agents, as well as with admixtures and combinations thereof.

## Patentansprüche

1. Überzogene Tablette, umfassend:
• einen Kern, umfassend:
∘ Refametinib, ein Hydrat, Solvat, Polymorph oder ein pharmazeutisch unbedenkliches Salz davon; und
∘ einen oder mehrere pharmazeutisch unbedenkliche Kernhilfsstoffe; und
• einen polyethylenglykolfreien, stabilitätsbewahrenden Überzug, umfassend:
∘ einen stabilitätsbewahrenden Filmbildner, bei dem es sich um Hydroxypropylmethylcellulose handelt;
∘ gegebenenfalls einen weiteren stabilitätsbewahrenden Filmbildner;
∘ und gegebenenfalls
∘ einen oder mehrere pharmazeutisch unbedenkliche Überzugshilfsstoffe.

2. Überzogene Tablette nach Anspruch 1, **dadurch gekennzeichnet, dass** der weitere stabilitätsbewahrende Filmbildner ausgewählt ist aus der Gruppe bestehend aus: Hydroxyethylcellulose, Hydroxypropylcellulose, Methylcellulose, Polyvinylalkohol (PVA), Polyvinylpyrrolidon, einem Copolymer aus Vinylpyrrolidon und Vinylacetat, einem Copolymer aus Vinylpyrrolidon und Vinylacetatsaccharose, Polyacrylat und Polymethacrylat, einem Copolymer aus Dimethylaminomethacrylisäure und einem neutralen Methacrylsäureester, einem Polymer von Methacrylsäure oder Methacrylsäureestern, einem Copolymer aus Acrylsäureethylester und Methacrylsäuremethylester, einem Copolymer aus Methacrylsäure und Acrylsäuremethylester, einem Copolymer aus Acrylsäure und Acrylsäuremethylester, flüssiger Glukose, Ethylcellulose, Celluloseacetatphthalat, Hydroxypropylmethylcelluloseacetatsuccinat, Hydroxypropylmethylcellulosephthalat, Schellack, und Mischungen davon, vorzugsweise Polyvinylalkohol (PVA).

3. Überzogene Tablette nach einem der Ansprüche 1 oder 2, **dadurch gekennzeichnet, dass** die Hydroxypropylmethylcellulose in einer Menge von 30 bis 100 Gew.-%, vorzugsweise 40 bis 55 Gew.-% des polyethylenglykolfreien, stabilitätsbewahrenden Überzugs vorhanden ist.

4. Überzogene Tablette, umfassend:
• einen Kern, umfassend:
∘ Refametinib, ein Hydrat, Solvat, Polymorph oder ein pharmazeutisch unbedenkliches Salz davon; und
∘ einen oder mehrere pharmazeutisch unbedenkliche Kernhilfsstoffe; und
• einen stabilitätsbewahrenden Überzug, umfassend:
∘ einen stabilitätsbewahrenden Filmbildner, bei dem es sich um Polyvinylalkohol, insbesondere hydrolysierten Polyvinylalkohol handelt;
∘ gegebenenfalls einen weiteren stabilitätsbewahrenden Filmbildner;
∘ einen Weichmacher, bei dem es sich um Polyethylenglycol handelt;
∘ und gegebenenfalls
∘ einen oder mehrere pharmazeutisch unbedenkliche Überzugshilfsstoffe.

5. Überzogene Tablette nach Anspruch 1 oder 4, **dadurch gekennzeichnet, dass** das Refametinib in mikronisierter Form vorliegt.

6. Überzogene Tablette nach einem der Ansprüche 1 oder 4, **dadurch gekennzeichnet, dass** das Refametinib in der Form von Polymorph A vorliegt.

7. Überzogene Tablette nach Anspruch 4, **dadurch gekennzeichnet, dass** es sich bei dem stabilitätsbewahrenden Filmbildner um Polyvinylalkohol (teilweise hydrolisiert) (PVA) handelt und dieser vorzugsweise in einer Menge von 40 bis 55 Gew.-% vom stabilitätsbewahrenden Überzug vorliegt.

8. Überzogene Tablette nach einem der Ansprüche 4 oder 7, **dadurch gekennzeichnet, dass** der weitere stabilitätsbewahrende Filmbildner ausgewählt ist aus der Gruppe bestehend aus: Hydroxyethylcellulose, Hydroxypropylcellulose, Methylcellulose, Polyvinylpyrrolidon, einem Copolymer aus Vinylpyrrolidon und Vinylacetat, einem Copolymer aus Vinylpyrrolidon und Vinylacetatsaccharose, Polyacrylat und Polymethacrylat, einem Copolymer aus Dimethylaminomethacrylisäure und einem neutralen Methacrylsäureester, einem Polymer von Methacrylsäure oder Methacrylsäureestern, einem Copolymer aus Acrylsäureethylester und Methacrylsäuremethylester, einem Copolymer aus Methacrylsäure und Acrylsäuremethylester, einem Copolymer aus Acrylsäure und Acrylsäuremethylester, flüssiger Glukose, Ethylcellulose, Celluloseacetatphthalat, Hydroxypropylmethylcelluloseacetatsuccinat, Hydroxypropylmethylcellulosephthalat, Schellack, und Mischungen davon.

9. Überzogene Tablette nach einem der Ansprüche 1 bis 8, **dadurch gekennzeichnet, dass** der eine oder die mehreren pharmazeutisch unbedenklichen, in dem stabilitätsbewahrenden Überzug vorhandenen Überzugshilfsstoffe ausgewählt ist (sind) aus der Gruppe bestehend aus:
* einem oder mehreren Weichmachern, ausgewählt aus der Gruppe bestehend aus: Propylenglycol, Polypropylenglycol, Sorbitol, Glycerol, Maltitol, Xylitol, Mannitol, Erythritol, Glyceroltrioleat, Tributylcitrat, Triethylcitrat, Acetyltriethylcitrat, Glyceryltriacetat, Stearinsäure, mittelkettige Triglyceride, und Mischungen davon;
* einem oder mehreren Farbstoffen, ausgewählt aus der Gruppe bestehend aus: Eisenoxidrot, Eisenoxidgelb, Eisenoxidschwarz, Titandioxid, Indigotin, Sunsetgelb FCF, Tartrazin, Erythrosin, Chinolingelb, Ruß, Anthocyan, Riboflavin, Karminrot, Curcumin, Chlorophyll, Carotin, und Mischungen davon, vorzugsweise Titandioxid und ein Eisenoxid, wie beispielsweise Eisenoxidgelb, Eisenoxidrot und/oder Eisenoxidschwarz, noch bevorzugter ein Eisenoxid und Titandioxid;
* einem oder mehreren Trennmitteln, ausgewählt aus der Gruppe bestehend aus: Talk, Magnesiumstearat, Stearinsäure, Lecithin, Sojalecithin, Mineralöl, Carnaubawachs, acetylierte Monoglyceride und/oder Polysorbat und Mischungen davon, vorzugsweise Talk, Lecithin, Sojalecithin und/oder Polysorbat, und Mischungen davon, noch bevorzugter Talk, Lecithin, Sojalecithin; und
* einem oder mehreren Trübungsmitteln, ausgewählt aus der Gruppe bestehend aus: Talk und Titandioxid.

10. Überzogene Tablette nach einem der Ansprüche 1 bis 9, **dadurch gekennzeichnet, dass** der eine oder die mehreren pharmazeutisch unbedenkliche(n), in dem Kern vorhandene(n) Kernhilfsstoff(e) ausgewählt ist (sind) aus der Gruppe bestehend aus:
* einem oder mehreren Füllstoffen oder Trockenbindemitteln, ausgewählt aus der Gruppe bestehend aus: Cellulosepulver, mikrokristalliner Cellulose, verkieselter mikrokristalliner Cellulose, Ethylcellulose, Dicalciumphosphat, Tricalciumphosphat, Kaolin, Magnesiumtrisilicat, Mannitol, Sorbitol, Maltitol, Xylitol, Lactose, Dextrose, Maltose, Saccharose, Glukose, Fruktose, Maltodextrine, gefälltem Calciumcarbonat, Natriumcarbonat, Natriumphosphat und Stärke, vorzugsweise mikrokristalliner Cellulose, Mannitol, und/oder Lactose, noch bevorzugter Mannitol, mikrokristalliner Cellulose und/oder Lactose;
* einem oder mehreren Sprengmitteln, ausgewählt aus der Gruppe bestehend aus: Croscarmellose-Natrium, Crospovidon (quervernetztes Polyvinylpyrrolidon), Natriumstärkeglycolat, Carboxymethylcellulose-Calcium, Carboxymethylcellulose-Natrium, mikrokristalliner Cellulose, Hydroxypropylcellulose, niedrig substituierter Hydroxypropylcellulose, Polacrilin-Kalium, Alginsäure, Natriumalginat, teilweise hydrolisierter Stärke, Natriumcarboxymethylstärke und Stärke, vorzugsweise Croscarmellose-Natrium und/oder Crospovidon, noch bevorzugter Croscarmellose-Natrium;
* einem oder mehreren Tensiden, insbesondere einem Benetzungsmittel, ausgewählt aus der Gruppe bestehend aus: Heptadecaethylenoxycetanol, Lecithinen, Polyoxyethylenstearat, Nonoxynol 9, Nonoxynol 10, Oxtoxynol 9, Sorbitanfettsäureestern, Polysorbaten, Natriumsalzen von Fettalkoholsulfaten, Natriumsalzen von Sulfosuccinaten, teilweise Estern von Fettsäuren mit Alkoholen, wie beispielsweise Glycerinmonostearat, Ethern von Fettalkoholen mit Polyoxyethylen, Estern von Fettsäuren mit Polyoxyethylen, Copolymeren aus Ethylenoxid und Propylenoxid (Pluronic®), Benzalkoniumchlorid und ethoxylierte Triglyceride, vorzugsweise Natriumlaurylsulfat;
* einem oder mehreren Bindemitteln (zur Nassgranulierung), ausgewählt aus der Gruppe bestehend aus: Hydroxypropylcellulose, Hypromellose (Hydroxypropylmethylcellulose, HPMC), Hydroxyethylcellulose, Carboxymethylcellulose, Carboxymethylcellulose-Calcium, Carboxymethylcellulose-Natrium, Ethylcellulose, Methylcellulose, Povidon (Polyvinylpyrrolidon, PVP), Polyvinylalkohol, Polyacrylaten, Gelatine, flüssiger Glucose, Gummi arabicum, Alginsäure und vorgelatinierte Stärke, vorzugsweise ein hydrophiles Bindemittel, das in der wässrigen Granulierungsflüssigkeit lösbar ist, noch bevorzugter Hypromellose (Hydroxypropylmethylcellulose, HPMC) und/oder Polyvinylpyrrolidon, ferner noch bevorzugter Hypromellose;
* einem oder mehreren Gleitmitteln, ausgewählt aus der Gruppe bestehend aus: Calciumstearat, Magnesiumstearat, Zinkstearat, Stearinsäure, Fumarsäure, Natriumstearylfumarat, Glyceroldibehenat, Glyceroldistearat, Glyceryldipalmitostearat, Behenoylpolyoxyl-8-Glyceriden, Mineralöl und Polyethylenglycol, vorzugsweise Magnesiumstearat.

11. Überzogene Tablette nach einem der Ansprüche 1, 2, 3, 9 oder 10, umfassend
• einen Kern, umfassend:
∘ Refametinib, bei dem es sich um mikronisiertes Polymorph A handelt; und
∘ pharmazeutisch unbedenkliche Kernhilfsstoffe, bei denen es sich um Folgende handelt:
• einen Füllstoff und ein Trockenbindemittel, bei dem es sich um Mannitol handelt, der vorzugsweise in einer Menge von 0 bis 95 Gew.-%, noch bevorzugter von 30 bis 80 Gew.-% vom Gesamtgewicht des Kerns vorhanden ist;
• ein Sprengmittel, bei dem es sich um Crocarmellose-Natrium handelt, das vorzugsweise in einer Menge von 0 bis 15 Gew.-%, noch bevorzugter von 3 bis 10 Gew.-% vom Gesamtgewicht des Kerns beinhaltet ist;
• ein Tensid, bei dem es sich um Natriumlaurylsulfat handelt, das vorzugsweise in einer Menge von 0,5 bis 1 Gew.-%, noch bevorzugter von 0,1 bis 2 Gew.-% vom Gesamtgewicht des Kerns vorhanden ist;
• ein Bindemittel, bei dem es sich um Hypromellose handelt, das vorzugsweise in einer Menge von 0 bis 15 Gew.-%, noch bevorzugter von 0,5 bis 8 Gew.-% vom Gesamtgewicht des Kerns vorhanden ist;
• ein Gleitmittel, bei dem es sich um Magnesiumstearat handelt, das vorzugsweise in einer Menge von 0 bis 5 Gew.-%, noch bevorzugter von 0,2 bis 3 Gew.-% vom Gesamtgewicht des Kerns beinhaltet ist;
und
• einen polyethylenglykolfreien, stabilitätsbewahrenden Überzug, umfassend:
∘ einen stabilitätsbewahrenden Filmbildner, bei dem es sich um Folgendes handelt:
▪ Hydroxypropylmethylcellulose (Hypromellose (HPMC)), die vorzugsweise in einer Menge von 30 bis 100 Gew.-%, noch bevorzugter von 40 bis 55 Gew.-% von dem polyethylenglykolfreien, stabilitätsbewahrenden Überzug vorhanden ist; und gegebenenfalls
∘ pharmazeutisch unbedenkliche Überzugshilfsstoffe, bei denen es sich um Folgende handelt:
• einen Farbstoff, bei dem es sich um ein Eisenoxid oder Titandioxid handelt, der vorzugsweise in einer Menge von 10 bis 50 Gew.-% von dem polyethylenglykolfreien, stabilitätsbewahrenden Überzug vorhanden ist;
• ein Trennmittel, bei dem es sich um Talk handelt, das vorzugsweise in einer Menge von 1 bis 40 Gew.-%, noch bevorzugter von 3 bis 25 Gew.-% von dem polyethylenglykolfreien, stabilitätsbewahrenden Überzug vorhanden ist; und
• ein Trübungsmittel, bei dem es sich um Talk und/oder Titandioxid handelt, das vorzugsweise in einer Menge von 10 bis 60 Gew.-% von dem polyethylenglykolfreien, stabilitätsbewahrenden Überzug vorhanden ist.

12. Überzogene Tablette nach einem der Ansprüche 4, 7, 9 oder 10, umfassend:
• einen Kern, umfassend:
∘ Refametinib, bei dem es sich um mikronisiertes Polymorph A handelt; und
∘ pharmazeutisch unbedenkliche Kernhilfsstoffe, bei denen es sich um Folgende handelt:
• einen Füllstoff und ein Trockenbindemittel, bei dem es sich um Mannitol handelt, der vorzugsweise in einer Menge von 0 bis 95 Gew.-%, noch bevorzugter von 30 bis 80 Gew.-% vom Gesamtgewicht des Kerns vorhanden ist;
• ein Sprengmittel, bei dem es sich um Crocarmellose-Natrium handelt, das vorzugsweise in einer Menge von 0 bis 15 Gew.-%, noch bevorzugter von 3 bis 10 Gew.-% vom Gesamtgewicht des Kerns beinhaltet ist;
• ein Tensid, bei dem es sich um Natriumlaurylsulfat handelt, das vorzugsweise in einer Menge von 0,5 bis 1 Gew.-%, noch bevorzugter von 0,1 bis 2 Gew.-% vom Gesamtgewicht des Kerns vorhanden ist;
• ein Bindemittel, bei dem es sich um Hypromellose handelt, das vorzugsweise in einer Menge von 0 bis 15 Gew.-%, noch bevorzugter von 0,5 bis 8 Gew.-% vom Gesamtgewicht des Kerns vorhanden ist;
• ein Gleitmittel, bei dem es sich um Magnesiumstearat handelt, das vorzugsweise in einer Menge von 0 bis 5 Gew.-%, noch bevorzugter von 0,2 bis 3 Gew.-% vom Gesamtgewicht des Kerns beinhaltet ist;
und
• einen stabilitätsbewahrenden Überzug, umfassend:
∘ einen stabilitätsbewahrenden Filmbildner, bei dem es sich um Folgendes handelt:
▪ Polyvinylalkohol (teilweise hydrolysiert) (PVA), der vorzugsweise in einer Menge von 30 bis 100 Gew.-%, noch bevorzugter von 40 bis 55 Gew.-% von dem stabilitätsbewahrenden Überzug vorhanden ist;
▪ Polyethylenglycol (PEG), das vorzugsweise in einer Menge von 0 bis 19 Gew.-% von dem stabilitätsbewahrenden Überzug vorhanden ist; und gegebenenfalls
∘ pharmazeutisch unbedenkliche Überzugshilfsstoffe, bei denen es sich um Folgende handelt:
• einen Farbstoff, bei dem es sich um ein Eisenoxid oder Titandioxid handelt, der vorzugsweise in einer Menge von 10 bis 50 Gew.-% von dem stabilitätsbewahrenden Überzug vorhanden ist;
• ein Trennmittel, bei dem es sich um Talk handelt, das vorzugsweise in einer Menge von 1 bis 40 Gew.-%, noch bevorzugter von 3 bis 25 Gew.-% von dem stabilitätsbewahrenden Überzug vorhanden ist; und
• ein Trübungsmittel, bei dem es sich um Talk und/oder Titandioxid handelt, das vorzugsweise in einer Menge von 10 bis 60 Gew.-% von dem stabilitätsbewahrenden Überzug vorhanden ist.

13. Überzogene Tablette nach einem der Ansprüche 1 bis 12, die 10, 20, 30 oder 50 mg Refametinib enthält.

14. Verfahren zum Herstellen einer überzogenen Tablette nach einem der Ansprüche 1 bis 13, das die folgenden Schritte umfasst:
a) Granulierung, wobei:
i) der Binder und das Benetzungsmittel in Wasser gegeben und gelöst werden, sodass eine Granulierflüssigkeit bereitgestellt wird;
ii) die Granulierflüssigkeit, wie zum Beispiel durch Sprühen, auf eine Mischung aus mikronisiertem Polymorph A Refametinib, dem Füllstoff/Trockenbindemittel und dem Sprengmittel, zum Beispiel als ein Pulverbett, zum Beispiel in einem Wirbelschichtgranulator, bei einer Temperatur von zum Beispiel 20 bis 35 °C, vorzugsweise von 28 bis 34 °C aufgetragen wird;
wodurch ein Refametinib enthaltendes Granulat bereitgestellt wird;
b) Tablettieren, wobei:
i) das Granulat auf eine geeignete Größe, wie zum Beispiel 0,5 bis 1,0 mm, vorzugsweise 0,8 mm, gesiebt wird, sodass ein gesiebtes, Refametinib enthaltendes Granulat bereitgestellt wird;
ii) Vermischen des gesiebten, Refametinib enthaltenden Granulats mit dem Sprengmittel und dem Gleitmittel, sodass eine pressfertige, Refametinib enthaltende Mischung bereitgestellt wird;
iii) Pressen der Refametinib enthaltenden Mischung in Tabletten, wie zum Beispiel auf einer Tabletten-Rundlaufpresse, sodass unüberzogene Refametinib enthaltende Tabletten bereitgestellt werden, die zum Beispiel 10, 20, 30 oder 50 mg Refametinib enthalten;
c) Filmüberzug, wobei:
i) der stabilitätsbewahrende Filmbildner, das Trennmittel und die Farbstoffe sowie gegebenenfalls der Weichmacher in Wasser dispergiert werden, sodass eine Überzugsdispersion bereitgestellt wird;
ii) die Überzugsdispersion auf die Refametinib enthaltenden unbüberzogenen Tabletten, zum Beispiel durch Sprühen aufgetragen wird, wie zum Beispiel in einer Siebtrommelbeschichtungsvorrichtung, zum Beispiel bei einer Temperatur von 35 bis 60 °C, vorzugsweise von 40 bis 55° C;
sodass überzogene Tabletten nach einem der Ansprüche 1 bis 13 bereitgestellt werden.

15. Überzogene Tablette nach einem der Ansprüche 1 bis 13 zur Verwendung bei der Behandlung oder Prophylaxe einer Erkrankung, insbesondere Krebs, wie beispielsweise hepatozellulärem Karzinom, Bauchspeicheldrüsenkrebs, Kolorektalkarzinom, nichtkleinzelligem Lungenkarzinom, Nicht-Hodgkin-Lymphom oder Brustkrebs; gegebenenfalls in Kombination mit einem oder mehreren Wirkstoffen, wie beispielsweise Mitteln gegen Hyperproliferation oder zytotoxischen Mitteln, Signaltransduktions-Inhibitoren oder mit anderen Antikrebsmitteln oder -therapien oder anderen Indikationsmitteln sowie mit Beimischungen und Kombinationen davon.

## Revendications

1. Comprimé enrobé comprenant :
• un coeur, comprenant :
∘ du réfamétinib, un hydrate, un solvate, un polymorphe ou un sel pharmaceutiquement acceptable de celui-ci ; et
∘ un ou plusieurs excipients de coeur pharmaceutiquement acceptables ; et
• un enrobage préservant la stabilité exempt de polyéthylène glycol, comprenant :
∘ un agent filmogène préservant la stabilité, qui est l'hydoxypropylméthylcellulose ;
∘ éventuellement, un agent filmogène supplémentaire préservant la stabilité ;
∘ et, éventuellement,
∘ un ou plusieurs excipients d'enrobage pharmaceutiquement acceptables.

2. Comprimé enrobé selon la revendication 1, **caractérisé en ce que** ledit agent filmogène supplémentaire préservant la stabilité est choisi dans le groupe constitué par : l'hydroxyéthylcellulose, l'hydroxypropylcellulose, la méthylcellulose, l'alcool polyvinylique (PVA), la polyvinylpyrrolidone, un copolymère de vinylpyrrolidone et de vinylacétate, un copolymère de vinylpyrrolidone et de saccharose de vinylacétate, le polyacrylate et le polyméthacrylate, un copolymère d'acide diméthylaminométhacrylique et d'un ester d'acide méthacrylique neutre, un polymère d'acide méthacrylique ou d'esters d'acide méthacrylique, un copolymère d'éthylester d'acide acrylique et de méthylester d'acide méthacrylique, un copolymère d'acide méthacrylique et de méthylester d'acide acrylique, un copolymère d'acide acrylique et de méthylester d'acide acrylique, le glucose liquide, l'éthylcellulose, le phtalate d'acétate de cellulose, le succinate d'acétate d'hydroxypropylméthylcellulose, le phtalate d'hydroxypropylméthylcellulose, la gomme laque, et des mélanges de ceux-ci, de préférence l'alcool polyvinylique (PVA).

3. Comprimé enrobé selon l'une quelconque des revendications 1 ou 2, **caractérisé en ce que** ladite hydroxypropylméthylcellulose est présente en une quantité représentant 30 à 100 %, de préférence 40 à 55 %, en poids dudit enrobage préservant la stabilité exempt de polyéthylène glycol.

4. Comprimé enrobé comprenant :
• un coeur, comprenant :
∘ du réfamétinib, un hydrate, un solvate, un polymorphe ou un sel pharmaceutiquement acceptable de celui-ci ; et
∘ un ou plusieurs excipients de coeur pharmaceutiquement acceptables ; et
• un enrobage préservant la stabilité, comprenant :
∘ un agent filmogène préservant la stabilité, qui est un alcool polyvinylique, en particulier un alcool polyvinylique partiellement hydrolysé ;
∘ éventuellement, un agent filmogène supplémentaire préservant la stabilité ;
∘ un plastifiant qui est le polyéthylène glycol ;
∘ et, éventuellement,
∘ un ou plusieurs excipients d'enrobage pharmaceutiquement acceptables.

5. Comprimé enrobé selon la revendication 1 ou 4, **caractérisé en ce que** ledit réfamétinib est sous forme micronisée.

6. Comprimé enrobé selon la revendication 1 ou 4, **caractérisé en ce que** ledit réfamétinib est sous la forme du polymorphe A.

7. Comprimé selon la revendication 4, **caractérisé en ce que** ledit agent filmogène préservant la stabilité étant un alcool polyvinylique (partiellement hydrolysé) (PVA) et étant présent en une quantité représentant 40 à 55 %, de préférence en poids dudit enrobage préservant la stabilité.

8. Comprimé enrobé selon l'une quelconque des revendications 4 ou 7, **caractérisé en ce que** ledit agent filmogène supplémentaire préservant la stabilité est choisi dans le groupe constitué par : l'hydroxyéthylcellulose, l'hydroxypropylcellulose, la méthylcellulose, la polyvinylpyrrolidone, un copolymère de vinylpyrrolidone et de vinylacétate, un copolymère de vinylpyrrolidone et de saccharose de vinylacétate, le polyacrylate et le polyméthacrylate, un copolymère d'acide diméthylaminométhacrylique et d'un ester d'acide méthacrylique neutre, un polymère d'acide méthacrylique ou d'esters d'acide méthacrylique, un copolymère d'éthylester d'acide acrylique et de méthylester d'acide méthacrylique, un copolymère d'acide méthacrylique et de méthylester d'acide acrylique, un copolymère d'acide acrylique et de méthylester d'acide acrylique, le glucose liquide, l'éthylcellulose, le phtalate d'acétate de cellulose, le succinate d'acétate d'hydroxypropylméthylcellulose, le phtalate d'hydroxypropylméthylcellulose, la gomme laque, et des mélanges de ceux-ci.

9. Comprimé enrobé selon l'une quelconque des revendications 1 à 8, **caractérisé en ce que** lesdits un ou plusieurs excipients d'enrobage pharmaceutiquement acceptables présents dans ledit enrobage préservant la stabilité sont choisis dans le groupe constitué par :
* un ou plusieurs plastifiants, choisis dans le groupe constitué par : le propylène glycol, polypropylène glycol, le sorbitol, le glycérol, le maltitol, le xylitol, le mannitol, l'érythritol, le trioléate de glycérol, le citrate de tributyle, le citrate de triéthyle, le triéthylcitrate d'acétyle, le triacétate de glycéryle, l'acide stéarique, les triglycérides à chaîne moyenne, et des mélanges de ceux-ci ;
* un ou plusieurs colorants choisis dans le groupe constitué par : le rouge d'oxyde ferrique, le jaune d'oxyde ferrique, le noir d'oxyde ferrique, le dioxyde de titane, l'indigotine, le jaune orangé FCF, la tartrazine, l'érythrosine, le jaune de quinoline, le noir de carbone, l'anthocyanine, la riboflavine, la carminé, la curcumine, la chlorophylle, le carotène et des mélanges de ceux-ci, de préférence le dioxyde de titane et un oxyde ferrique, tel que le jaune d'oxyde ferrique, le rouge d'oxyde ferrique et/ou le noir d'oxyde ferrique, plus préférablement un oxyde ferrique et le dioxyde de titane ;
* un ou plusieurs agents antiadhérents choisis dans le groupe constitué par : le talc, le stéarate de magnésium, l'acide stéarique, la lécithine, la lécithine de soja, une huile minérale, la cire de carnauba, les monoglycérides acétylés et/ou le polysorbate et des mélanges de ceux-ci, de préférence le talc, la lécithine, la lécithine de soja et/ou le polysorbate, et des mélanges de ceux-ci, plus préférablement le talc, la lécithine et la lécithine de soja ; et
* un ou plusieurs opacifiants choisis dans le groupe constitué par : le talc et le dioxyde de titane.

10. Comprimé enrobé selon l'une quelconque des revendications 1 à 9, **caractérisé en ce que** lesdits un ou plusieurs excipients de coeur pharmaceutiquement acceptables présents dans ledit coeur sont choisis dans le groupe constitué par :
* un(e) ou plusieurs charges ou liants secs choisi(e)s dans le groupe constitué par : la cellulose en poudre, la cellulose microcristalline, la cellulose microcristalline silicifiée, l'éthylcellulose, le phosphate dicalcique, le phosphate tricalcique, le kaolin, le trisilicate de magnésium, le mannitol, le sorbitol, le maltitol, le xylitol, le lactose, le dextrose, le maltose, le saccharose, le glucose, le fructose, les maltodextrines, le carbonate de calcium précipité, le carbonate de sodium, le phosphate de sodium et l'amidon, de préférence la cellulose microcristalline, le mannitol et/ou le lactose, plus préférablement le mannitol, la cellulose microcristalline et/ou le lactose ;
* un ou plusieurs délitants choisis dans le groupe constitué par : la croscarmellose sodique, la crospovidone (polyvinylpyrrolidone réticulée), le glycolate d'amidon de sodium, la carboxyméthylcellulose calcique, la carboxyméthylcellulose sodique, la cellulose microcristalline, l'hydroxypropylcellulose, l'hydroxypropylcellulose faiblement substituée, la polacriline potassique, l'acide alginique, l'alginate de sodium, l'amidon partiellement hydrolysé, le carboxyméthylamidon sodique et l'amidon, de préférence la croscarmellose sodique et/ou la crospovidone, plus préférablement la croscarmellose sodique ;
* un ou plusieurs tensioactifs, en particulier un agent mouillant choisi dans le groupe constitué par : l'heptadécaéthylène oxycétanol, les lécithines, le stéarate de polyoxyéthylène, le nonoxynol 9, le nonoxynol 10, l'oxtoxynol 9, les esters d'acides gras de sorbitane, les polysorbates, les sels sodiques de sulfates d'acides gras, les sels sodiques de sulfosuccinates, les esters partiels d'acides gras avec des alcools tels que le monostéarate de glycérine, les éthers d'alcools gras avec du polyoxyéthylène, les esters d'acides gras avec du polyoxyéthylène, les copolymères d'oxyde d'éthylène et d'oxyde de propylène (Pluronic®), le chlorure de benzalkonium et les triglycérides éthoxylées, de préférence le laurylsulfate de sodium ;
* un ou plusieurs liants (pour la granulation humide) choisis dans le groupe constitué par : l'hydroxypropylcellulose, l'hypromellose (hydroxypropylméthylcellulose, HPMC), l'hydroxyéthylcellulose, la carboxyméthylcellulose, la carboxyméthylcellulose calcique, la carboxyméthylcellulose sodique, l'éthylcellulose, la méthylcellulose, la povidone (polyvinylpyrrolidone, PVP), l'alcool polyvinylique, les polyacrylates, la gélatine, le glucose liquide, la gomme arabique, l'acide alginique et l'amidon prégélatinisé, de préférence un liant hydrophile qui est soluble dans le liquide de granulation aqueux, plus préférablement l'hypromellose (hydroxypropylméthylcellulose, HPMC) et/ou la polyvinylpyrrolidone, plus préférablement encore l'hypromellose ;
* un ou plusieurs lubrifiants choisis dans le groupe constitué par : le stéarate de calcium, le stéarate de magnésium, le stéarate de zinc, l'acide stéarique, l'acide fumarique, le stéarylfumarate de sodium, le dibéhénate de glycérol, le distéarate de glycérol, dipalmitostéarate de glycéryle, les glycérides de polyoxyl-8 de béhénoyle, une huile minérale et le polyéthylène glycol, de préférence le stéarate de magnésium.

11. Comprimé enrobé selon l'une quelconque des revendications 1, 2, 3, 9 ou 10, qui comprend :
• un coeur, comprenant :
∘ du réfamétinib, qui est le polymorphe A micronisé ; et
∘ des excipients de coeur pharmaceutiquement acceptables, qui sont :
• une charge et un liant sec, qui est le mannitol, présent de préférence en une quantité représentant 0 à 95 %, plus préférablement 30 à 80 %, en poids du poids total dudit coeur ;
• un délitant qui est la crocarmellose sodique, contenu de préférence en une quantité représentant 0 à 15 %, plus préférablement 3 à 10 %, en poids du poids total dudit coeur ;
• un tensioactif qui est le laurylsulfate de sodium, présent de préférence en une quantité représentant 0,5 à 1 %, plus préférablement 0,1 à 2 %, en poids du poids total dudit coeur ;
• un liant, qui est l'hypromellose, présent de préférence en une quantité représentant 0 à 15 %, plus préférablement 0,5 à 8 %, en poids du poids total dudit coeur ;
• un lubrifiant, qui est le stéarate de magnésium, contenu de préférence en une quantité représentant 0 à 5 %, plus préférablement 0,2 à 3 %, en poids du poids total dudit coeur ;
et
• un enrobage préservant la stabilité exempt de polyéthylène glycol, comprenant :
∘ un agent filmogène préservant la stabilité, qui est :
▪ l'hydroxypropylméthylcellulose (hypromellose (HPMC)), de préférence présente en une quantité représentant 30 à 100 %, plus préférablement 40 à 55 %, en poids dudit revêtement préservant la stabilité exempt de polyéthylène glycol ; et, éventuellement,
∘ des excipients d'enrobage pharmaceutiquement acceptables, qui sont :
• un colorant, qui est un oxyde ferrique ou le dioxyde de titane, présent de préférence en une quantité représentant 10 à 50 % en poids dudit enrobage préservant la stabilité exempt de polyéthylène glycol ;
• un agent antiadhérent, qui est le talc, présent de préférence en une quantité représentant 1 à 40 %, plus préférablement 3 à 25 %, en poids dudit enrobage préservant la stabilité exempt de polyéthylène glycol ; et
• un opacifiant, qui est le talc et/ou le dioxyde de titane, présent de préférence en une quantité représentant 10 à 60 % en poids dudit enrobage préservant la stabilité exempt de polyéthylène glycol.

12. Comprimé enrobé selon l'une quelconque des revendications 4, 7, 9 ou 10, qui comprend :
• un coeur, comprenant :
∘ du réfamétinib, qui est le polymorphe A micronisé ; et
∘ des excipients de coeur pharmaceutiquement acceptables, qui sont :
• une charge et un liant sec, qui est le mannitol, présent de préférence en une quantité représentant 0 à 95 %, plus préférablement 30 à 80 %, en poids du poids total dudit coeur ;
• un délitant, qui est la crocarmellose sodique, contenu de préférence en une quantité représentant 0 à 15 %, plus préférablement 3 à 10 %, en poids du poids total dudit coeur ;
• un tensioactif, qui est le laurylsulfate de sodium, présent de préférence en une quantité représentant 0,5 à 1 %, plus préférablement 0,1 à 2 %, en poids du poids total dudit coeur ;
• un liant, qui est l'hypromellose, présent de préférence en une quantité représentant 0 à 15 %, plus préférablement 0,5 à 8 %, en poids du poids total dudit coeur ;
• un lubrifiant, qui est le stéarate de magnésium, contenu de préférence en une quantité représentant 0 à 5 %, plus préférablement 0,2 à 3 %, en poids du poids total dudit coeur ;
et
• un enrobage préservant la stabilité, comprenant :
∘ un agent filmogène préservant la stabilité, qui est :
▪ l'alcool polyvinylique (partiellement hydrolysé) (PVA), présent de préférence en une quantité représentant 30 à 100 %, plus préférablement 40 à 55 %, en poids dudit enrobage préservant la stabilité ;
▪ le polyéthylène glycol (PEG), présent de préférence en une quantité représentant 0 à 19 % en poids dudit enrobage préservant la stabilité ;
et, éventuellement,
∘ des agents d'enrobage pharmaceutiquement acceptables, qui sont :
• un colorant, qui est un oxyde ferrique ou du dioxyde de titane, présent de préférence en une quantité représentant 10 à 50 % en poids dudit enrobage préservant la stabilité ;
• un agent antiadhérent, qui est le talc, présent de préférence en une quantité représentant 1 à 40 %, plus préférablement 3 à 25 %, en poids dudit enrobage préservant la stabilité ; et
• un opacifiant, qui est le talc et/ou le dioxyde de titane, présent de préférence en une quantité représentant 10 à 60 % en poids dudit enrobage préservant la stabilité.

13. Comprimé enrobé selon l'une quelconque des revendications 1 à 12, qui contient 10, 20, 30 ou 50 mg de réfamétinib.

14. Procédé de préparation d'un comprimé enrobé selon l'une quelconque des revendications 1 à 13, comprenant les étapes suivantes :
a) granulation, dans laquelle :
i) ledit liant et ledit agent mouillant sont mis dans de l'eau et dissous, assurant ainsi une granulation liquide ;
ii) ledit liquide de granulation est appliqué, tel que par pulvérisation par exemple, sur un mélange du polymorphe A micronisé de réfamétinib, ladite charge/ledit liant sec et ledit délitant, sous forme de lit pulvérulent par exemple, dans un granulateur à lit fluidisé par exemple, à une température telle qu'une température de 20 à 35°C par exemple, de préférence de 28 à 34°C ;
ce qui fournit un granulat contenant du réfamétinib ;
b) pastillage, dans lequel :
i) ledit granulat est criblé à une taille appropriée, telle que 0,5 à 1,0 mm par exemple, de préférence 0,8 mm, ce qui fournit un granulat criblé contenant du réfamétinib ;
ii) mélange dudit granulat criblé contenant du réfamétinib avec ledit délitant et ledit lubrifiant, ce qui fournit un mélange contenant du réfamétinib prêt à être comprimé ;
iii) compression dudit mélange contenant du réfamétinib en comprimés, telle que sur une presse à comprimés rotative par exemple, ce qui fournit des comprimés non enrobés contenant du réfamétinib, contenant 10, 20, 30 ou 50 mg de réfamétinib, par exemple ;
c) enrobage par film, dans lequel :
i) dispersion dudit agent filmogène préservant la stabilité, ledit agent antiadhérent et lesdits colorants ainsi que lesdits plastifiants éventuels dans l'eau, ce qui fournit une dispersion d'enrobage ;
ii) application, telle que par pulvérisation par exemple, de ladite dispersion d'enrobage sur lesdits comprimés non enrobés contenant du réfamétinib, telle que dans une enrobeuse à tambour perforé par exemple, à une température de 35 à 60°C par exemple, de préférence de 40 à 55°C ;
ce qui fournit des comprimés enrobés selon l'une quelconque des revendications 1 à 13.

15. Comprimé enrobé selon l'une quelconque des revendications 1 à 13 destiné à être utilisé dans le traitement ou la prophylaxie d'une maladie, en particulier un cancer, tel qu'un carcinome hépatocellulaire, le cancer du pancréas, le cancer colorectal, un carcinome pulmonaire non à petites cellules, un lymphome non hodgkinien ou le cancer du sein ; éventuellement en combinaison avec un ou plusieurs principes actifs, tels que des agents anti-hyperpolifératifs ou cytotoxiques, des inhibiteurs de transduction de signal, ou avec d'autres agents ou traitements anticancéreux, ou d'autres agents indicateurs, ainsi qu'avec des mélanges ou des combinaisons de ceux-ci.
